# EUROPEAN PATENT APPLICATION

(11) **EP 1 518 927 A2**
(43) Date of publication of application: **30.03.2005**
(21) Application number: 04078085.0
(22) Date of filing: 10.02.1999
(51) Int. Cl.: C12N 15/10, C07K 14/705, C07K 14/54, C07K 14/02, C12N 5/10, G01N 33/566, A61K 48/00

(54) **Optimization of immunomodulatory properties of genetic vaccines**

(30) Priority: 11.02.1998 US 74294 P; 11.02.1998 US 21769
(62) Divisional of application: 99906948.7
(71) Applicant: Maxygen, Inc., Redwood City, CA 94063 (US)
(72) Inventor: Punnonen, Juha, Belmont, CA 94002 (US); Stemmer, Willem P.C., Los Gatos, CA 95030 (US); Whalen, Robert Gerald, Foster City, CA 94404 (US); Howard, Russell J., Los Altos, CA 94022 (US)
(74) Representative: Salka, Jeffrey

(57) **Abstract**

This invention provides methods for obtaining molecules that can modulate an immune response, and immunomodulatory molecules obtained using the methods. The molecules find use, for example, in the tailoring of an immune response induced by a genetic vaccine for a desired purpose.

## Description

### STATEMENT REGARDING GOVERNMENT SUPPORT

This invention was made with Government support under Grant No. N65236-98-1-5401, awarded by the Defense Advanced Projects Agency. The Government has certain rights in this invention.

### BACKGROUND OF THE INVENTION

### Field of the Invention

This invention pertains to the field of modulation of immune responses such as those induced by genetic vaccines.

### Background

Antigen processing and presentation is only one factor which determines the effectiveness of vaccination, whether performed with genetic vaccines or more classical methods. Other molecules involved in determining vaccine effectiveness include cytokines (interleukins, interferons, chemokines, hematopoietic growth factors, tumor necrosis factors and transforming growth factors), which are small molecular weight proteins that regulate maturation, activation, proliferation and differentiation of the cells of the immune system. Characteristic features of cytokines are pleiotropy and redundancy; that is, one cytokine often has several functions and a given function is often mediated by more than one cytokine. In addition, several cytokines have additive or synergistic effects with other cytokines, and a number of cytokines also share receptor components.

Due to the complexity of the cytokine networks, studies on the physiological significance of a given cytokine have been difficult, although recent studies using cytokine gene-deficient mice have significantly improved our understanding on the functions of cytokines *in vivo.* In addition to soluble proteins, several membrane-bound costimulatory molecules play a fundamental role in the regulation of immune responses. These molecules include CD40, CD40 ligand, CD27, CD80, CD86 and CD150 (SLAM), and they are typically expressed on lymphoid cells after activation via antigen recognition or through cell-cell interactions.

T helper (T_{H}) cells, key regulators of the immune system, are capable of producing a large number of different cytokines, and based on their cytokine synthesis pattern T_{H} cells are divided into two subsets (Paul and Seder (1994) *Cell* 76: 241-251). T_{H}1 cells produce high levels of IL-2 and IFN-γ and no or minimal levels of IL-4, IL-5 and IL-13. In contrast, T_{H}2 cells produce high levels of IL-4, IL-5 and IL-13, and IL-2 and IFN-γ production is minimal or absent. T_{H}1 cells activate macrophages, dendritic cells and augment the cytolytic activity of CD8⁺ cytotoxic T lymphocytes and NK cells (*Id*.)*,* whereas T_{H}2 cells provide efficient help for B cells and they also mediate allergic responses due to the capacity of T_{H}2 cells to induce IgE isotype switching and differentiation of B cells into IgE secreting cell (De Vries and Punnonen (1996) In *Cytokine regulation of humoral immunity: basic and clinical aspects.* Eds. Snapper, C.M., John Wiley & Sons, Ltd., West Sussex, UK, p. 195-215). The exact mechanisms that regulate the differentiation ofT helper cells are not fully understood, but cytokines are believed to play major role. IL-4 has been shown to direct T_{H}2 differentiation, whereas IL-12 induces development of T_{H}1 cells (Paul and Seder, *supra*.). In addition, it has been suggested that membrane bound costimulatory molecules, such as CD80, CD86 and CD150, can direct T_{H}1 and/or T_{H}2 development, and the same molecules that regulate T_{H} cell differentiation also affect activation, proliferation and differentiation of B cells into Ig-secreting plasma cells (Cocks *et al.* (1995) *Nature* 376: 260-263; Lenschow *et al.* (1996) *Immunity* 5: 285-293; Punnonen *et al.* (1993) *Proc. Nat'l. Acad. Sci. USA.* 90: 3730-3734; Punnonen *et al.* (1997) *J. Exp. Med.* 185: 993-1004).

Studies in both man and mice have demonstrated that the cytokine synthesis profile of T helper (T_{H}) cells plays a crucial role in determining the outcome of several viral, bacterial and parasitic infections. High frequency of T_{H}1 cells generally protects from lethal infections, whereas dominant T_{H}2 phenotype often results in disseminated, chronic infections. For example, T_{H}1 phenotype is observed in tuberculoid (resistant) form of leprosy and T_{H}2 phenotype in lepromatous, multibacillary (susceptible) lesions (Yamamura *et al.* (1991) *Science* 254: 277-279). Similarly, late-stage HIV patients have T_{H}2-like cytokine synthesis profiles, and T_{H}1 phenotype has been proposed to protect from AIDS (Maggi *et al.* (1994) *J. Exp. Med.* 180: 489-495). Furthermore, the survival from meningococcal septicemia is genetically determined based on the capacity of peripheral blood leukocytes to produce TNF-α and IL-10. Individuals from families with high production of IL-10 have increased risk of fatal meningococcal disease, whereas members of families with high TNF-α production were more likely to survive the infection (Westendorp *et al.* (1997) *Lancet* 349: 170-173).

Cytokine treatments can dramatically influence T_{H}1/T_{H}2 cell differentiation and macrophage activation, and thereby the outcome of infectious diseases. For example, BALB/c mice infected with *Leishmania major* generally develop a disseminated fatal disease with a T_{H}2 phenotype, but when treated with anti-IL-4 mAbs or IL-12, the frequency of T_{H}1 cells in the mice increases and they are able to counteract the pathogen invasion (Chatelain *et al.* (1992) *J. Immunol.* 148: 1182-1187). Similarly, IFN-γ protects mice from lethal Herpes Simplex Virus (HSV) infection, and MCP-1 prevents lethal infections by *Pseudomonas aeruginosa* or *Salmonella typhimurium.* In addition, cytokine treatments, such as recombinant IL-2, have shown beneficial effects in human common variable immunodeficiency (Cunningham-Rundles *et al.* (1994) *N. Engl. J. Med.* 331: 918-921).

The administration of cytokines and other molecules to modulate immune responses in a manner most appropriate for treating a particular disease can provide a significant tool for the treatment of disease. However, presently available immunomodulator treatments can have several disadvantages, such as insufficient specific activity, induction of immune responses against, the immunomodulator that is administered, and other potential problems. Thus, a need exists for immunomodulators that exhibit improved properties relative to those currently available. The present invention fulfills this and other needs.

### SUMMARY OF THE INVENTION

The present invention provides methods of obtaining a polynucleotide that has a modulatory effect on an immune response that is induced by a genetic vaccine, either directly (*i.e.,* as an immunomodulatory polynucleotide) or indirectly (*i.e.,* upon translation of the polynucleotide to create an immunomodulatory polypeptide. The methods of the invention involve: creating a library of recombinant polynucleotides; and screening the library to identify at least one optimized recombinant polynucleotide that exhibits, either by itself or through the encoded polypeptide, an enhanced ability to modulate an immune response than a form of the nucleic acid from which the library was created. Examples include, for example, CpG-rich polynucleotide sequences, polynucleotide sequences that encode a costimulator (*e.g.*, B7-1, B7-2, CD1, CD40, CD154 (ligand for CD40), CD150 (SLAM), or a cytokine. The screening step used in these methods can include, for example, introducing genetic vaccine vectors which comprise the library of recombinant nucleic acids into a cell, and identifying cells which exhibit an increased ability to modulate an immune response of interest or increased ability to express an immunomodulatory molecule. For example, a library of recombinant cytokine-encoding nucleic acids can be screened by testing the ability of cytokines encoded by the nucleic acids to activate cells which contain a receptor for the cytokine. The receptor for the cytokine can be native to the cell, or can be expressed from a heterologous nucleic acid that encodes the cytokine receptor. For example, the optimized costimulators can be tested to identify those for which the cells or culture medium are capable of inducing a predominantly T_{H}2 immune response, or a predominantly T_{H}1 immune response.

In some embodiments, the polynucleotide that has a modulatory effect on an immune response is obtained by: (1) recombining at least first and second forms of a nucleic acid that is, or encodes a molecule that is, involved in modulating an immune response, wherein the first and second forms differ from each other in two or more nucleotides, to produce a library of recombinant polynucleotides; and (2) screening the library to identify at least one optimized recombinant polynucleotide that exhibits, either by itself or through the encoded polypeptide, an enhanced ability to modulate an immune response than a form of the nucleic acid from which the library was created. If additional optimization is desired, the method can further involve: (3) recombining at least one optimized recombinant polynucleotide with a further form of the nucleic acid, which is the same or different from the first and second forms, to produce a further library of recombinant polynucleotides; (4) screening the further library to identify at least one further optimized recombinant polynucleotide that exhibits an enhanced ability to modulate an immune response than a form of the nucleic acid from which the library was created.; and (5) repeating (3) and (4), as necessary, until the further optimized recombinant polynucleotide exhibits an further enhanced ability to modulate an immune response than a form of the nucleic acid from which the library was created.

In some embodiments of the invention, the library of recombinant polynucleotides is screened by: expressing the recombinant polynucleotides so that the encoded peptides or polypeptides are produced as fusions with a protein displayed on the surface of a replicable genetic package; contacting the replicable genetic packages with a plurality of cells that display the receptor; and identifying cells that exhibit a modulation of an immune response mediated by the receptor.

The invention also provides methods for obtaining a polynucleotide that encodes an accessory molecule that improves the transport or presentation of antigens by a cell. These methods involve creating a library of recombinant polynucleotides by subjecting to recombination nucleic acids that encode all or part of the accessory molecule; and screening the library to identify an optimized recombinant polynucleotide that encodes a recombinant accessory molecule that confers upon a cell an increased or decreased ability to transport or present an antigen on a surface of the cell compared to an accessory molecule encoded by the non-recombinant nucleic acids. In some embodiments, the screening step involves: introducing the library of recombinant polynucleotides into a genetic vaccine vector that encodes an antigen to form a library of vectors; introducing the library of vectors into mammalian cells; and identifying mammalian cells that exhibit increased or decreased immunogenicity to the antigen.

In some embodiments of the invention, the cytokine that is optimized is interleukin-12 and the screening is performed by growing mammalian cells which contain the genetic vaccine vector in a culture medium, and detecting whether T cell proliferation or T cell differentiation is induced by contact with the culture medium. In another embodiment, the cytokine is interferon-α and the screening is performed by expressing the recombinant vector module as a fusion protein which is displayed on the surface of a bacteriophage to form a phage display library, and identifying phage library members which are capable of inhibiting proliferation of a B cell line. Another embodiment utilizes B7-1 (CD80) or B7-2 (CD86) as the costimulator and the cell or culture medium is tested for ability to modulate an immune response.

The invention provides methods of using DNA shuffling to obtain optimized recombinant vector modules that encode cytokines and other costimulators that exhibit reduced immunogenicity compared to a corresponding polypeptide encoded by a non-optimized vector module. The reduced immunogenicity can be detected by introducing a cytokine or costimulator encoded by the recombinant vector module into a mammal and determining whether an immune response is induced against the cytokine.

The invention also provides methods of obtaining optimized immunomodulatory sequences that encode a cytokine antagonist. For example, suitable cytokine agonists include a soluble cytokine receptor and a transmembrane cytokine receptor having a defective signal sequence. Examples include ΔIL-10R and ΔIL-4R, and the like.

### BRIEF DESCRIPTION OF THE DRAWINGS

Figure 1 shows an example of a cytotoxic T-cell inducing sequence (CTIS) obtained from HBsAg polypeptide (PreS2 plus S regions).
Figure 2 shows a CTIS having heterologous epitopes attached to the cytoplasmic portion.
Figure 3 shows the derivation of immunogenic agonistic sequences (IAS) as described in Example 3. Specific killing (percent) is shown for an effector: target (E:T) ratio of five.
Figure 4 shows a method for preparing immunogenic agonist sequences (IAS). Wild-type (WT) and mutated forms of nucleic acids encoding a polypeptide of interest are assembled and subjected to DNA shuffling to obtain a nucleic acid encoding a poly-epitope region that contains potential agonist sequences.
Figure 5 shows a scheme for improving immunostimulatory sequences by DNA shuffling.
Figure 6 is a diagram of a procedure by which recombinant libraries of human IL-12 genes can be screened to identify shuffled IL-12 genes that encode recombinant IL-12 having increased ability to induce T cell proliferation.
Figure 7 shows the results of a high-throughput functional assay for vectors that encode variants of IL-12 obtained using the methods of the invention.
Figure 8 shows the induction of T cell proliferation upon transfection of the T cells by individual vectors that encode IL-12 variants.
Figure 9 shows results of an experiment which demonstrates that a shuffled IL-12 chimera obtained using the methods of the invention exhibits improved ability to activate human T cells.
Figure 10 shows a model of how T cell activation or anergy can be induced by genetic vaccine vectors that encode different B7-1 (CD80) and/or B7-2 (CD86) variants.
Figure 11 shows a method for using DNA shuffling to obtain CD80/CD86 variants that have improved capacity to induce T cell activation or anergy.

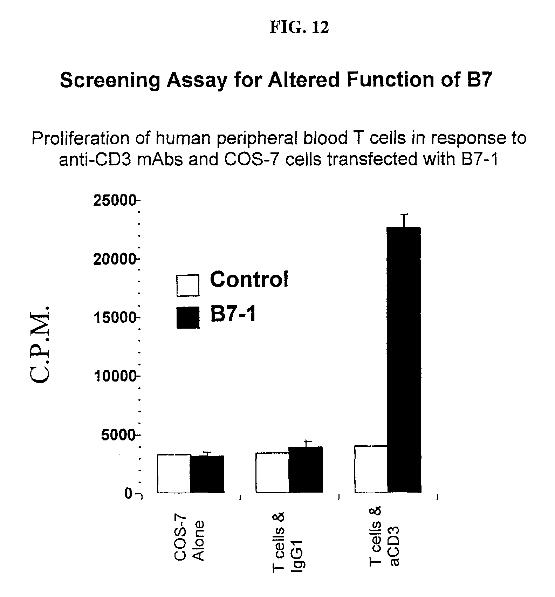

Figure 12 shows results obtained in a screening assay for altered function of B7.
Figure 13 provides experimental results which demonstrate that shuffled B7 chimeras provide potent T cell activation.
Figure 14 presents an alignment of the nucleotide sequences for human and mouse IL-10 receptor sequences.
Figure 15 shows an alignment of the nucleotide sequences of B7-1 (CD80) genes from human, rhesus monkey, and rabbit.

### DETAILED DESCRIPTION

### Definitions

The term "cytokine" includes, for example, interleukins, interferons, chemokines, hematopoietic growth factors, tumor necrosis factors and transforming growth factors. In general these are small molecular weight proteins that regulate maturation, activation, proliferation and differentiation of the cells of the immune system.

The term "screening" describes, in general, a process that identifies optimal immunomodulatory molecules. Several properties ofthe respective molecules can be used in selection and screening including, for example, ability to induce a desired immune response in a test system. Selection is a form of screening in which identification and physical separation are achieved simultaneously by expression of a selection marker, which, in some genetic circumstances, allows cells expressing the marker to survive while other cells die (or vice versa). Screening markers include, for example, luciferase, beta-galactosidase and green fluorescent protein. Selection markers include drug and toxin resistance genes, and the like. Because of limitations in studying primary immune responses *in vitro, in vivo* studies are particularly useful screening methods. In these studies, the genetic vaccines that include immunomodulatory molecules are first introduced to test animals, and the immune responses are subsequently studied by analyzing protective immune responses or by studying the quality or strength of the induced immune response using lymphoid cells derived from the immunized animal. Although spontaneous selection can and does occur in the course of natural evolution, in the present methods selection is performed by man.

A "exogenous DNA segment", "heterologous sequence" or a "heterologous nucleic acid", as used herein, is one that originates from a source foreign to the particular host cell, or, if from the same source, is modified from its original form. Thus, a heterologous gene in a host cell includes a gene that is endogenous to the particular host cell, but has been modified. Modification of a heterologous sequence in the applications described herein typically occurs through the use of DNA shuffling. Thus, the terms refer to a DNA segment which is foreign or heterologous to the cell, or homologous to the cell but in a position within the host cell nucleic acid in which the element is not ordinarily found. Exogenous DNA segments are expressed to yield exogenous polypeptides.

The term "gene" is used broadly to refer to any segment of DNA associated with a biological function. Thus, genes include coding sequences and/or the regulatory sequences required for their expression. Genes also include nonexpressed DNA segments that, for example, form recognition sequences for other proteins. Genes can be obtained from a variety of sources, including cloning from a source of interest or synthesizing from known or predicted sequence information, and may include sequences designed to have desired parameters.

The term "isolated", when applied to a nucleic acid or protein, denotes that the nucleic acid or protein is essentially free of other cellular components with which it is associated in the natural state. It is preferably in a homogeneous state although it can be in either a dry or aqueous solution. Purity and homogeneity are typically determined using analytical chemistry techniques such as polyacrylamide gel electrophoresis or high performance liquid chromatography. A protein which is the predominant species present in a preparation is substantially purified. In particular, an isolated gene is separated from open reading frames which flank the gene and encode a protein other than the gene of interest. The term "purified" denotes that a nucleic acid or protein gives rise to essentially one band in an electrophoretic get. Particularly, it means that the nucleic acid or protein is at least about 50% pure, more preferably at least about 85% pure, and most preferably at least about 99% pure.

The term "naturally-occurring" is used to describe an object that can be found in nature as distinct from being artificially produced by man. For example, a polypeptide or polynucleotide sequence that is present in an organism (including viruses, bacteria, protozoa, insects, plants or mammalian tissue) that can be isolated from a source in nature and which has not been intentionally modified by man in the laboratory is naturally-occurring.

The term "nucleic acid" refers to deoxyribonucleotides or ribonucleotides and polymers thereof in either single- or double-stranded form. Unless specifically limited, the term encompasses nucleic acids containing known analogues of natural nucleotides which have similar binding properties as the reference nucleic acid and are metabolized in a manner similar to naturally occurring nucleotides. Unless otherwise indicated, a particular nucleic acid sequence also implicitly encompasses conservatively modified variants thereof (*e.g.* degenerate codon substitutions) and complementary sequences and as well as the sequence explicitly indicated. Specifically, degenerate codon substitutions may be achieved by generating sequences in which the third position of one or more selected (or all) codons is substituted with mixed-base and/or deoxyinosine residues (Batzer *et al.* (1991) *Nucleic Acid Res. 19:* 5081; Ohtsuka *et al.* (1985) *J. Biol. Chem. 260:* 2605-2608; Cassol *et al.* (1992); Rossolini *et al.* (1994) *Mol. Cell. Probes 8*: 91-98). The term nucleic acid is used interchangeably with gene, cDNA, and mRNA encoded by a gene.

"Nucleic acid derived from a gene" refers to a nucleic acid for whose synthesis the gene, or a subsequence thereof, has ultimately served as a template. Thus, an mRNA, a cDNA reverse transcribed from an mRNA, an RNA transcribed from that cDNA, a DNA amplified from the cDNA, an RNA transcribed from the amplified DNA, *etc.,* are all derived from the gene and detection of such derived products is indicative of the presence and/or abundance of the original gene and/or gene transcript in a sample.

A nucleic acid is "operably linked" when it is placed into a functional relationship with another nucleic acid sequence. For instance, a promoter or enhancer is operably linked to a coding sequence if it increases the transcription of the coding sequence. Operably linked means that the DNA sequences being linked are typically contiguous and, where necessary to join two protein coding regions, contiguous and in reading frame. However, since enhancers generally function when separated from the promoter by several kilobases and intronic sequences may be of variable lengths, some polynucleotide elements may be operably linked but not contiguous.

A specific binding affinity between two molecules, for example, a ligand and a receptor, means a preferential binding of one molecule for another in a mixture of molecules. The binding of the molecules can be considered specific if the binding affinity is about 1 x 10⁴ M ⁻¹ to about 1 x 10⁶ M ⁻¹ or greater.

The term "recombinant" when used with reference to a cell indicates that the cell replicates a heterologous nucleic acid, or expresses a peptide or protein encoded by a heterologous nucleic acid. Recombinant cells can contain genes that are not found within the native (non-recombinant) form of the cell. Recombinant cells can also contain genes found in the native form of the cell wherein the genes are modified and re-introduced into the cell by artificial means. The term also encompasses cells that contain a nucleic acid endogenous to the cell that has been modified without removing the nucleic acid from the cell; such modifications include those obtained by gene replacement, site-specific mutation, and related techniques.

A "recombinant expression cassette" or simply an "expression cassette" is a nucleic acid construct, generated recombinantly or synthetically, with nucleic,acid elements that are capable of effecting expression of a structural gene in hosts compatible with such sequences. Expression cassettes include at least promoters and optionally, transcription termination signals. Typically, the recombinant expression cassette includes a nucleic acid to be transcribed (*e.g.*, a nucleic acid encoding a desired polypeptide), and a promoter, Additional factors necessary or helpful in effecting expression may also be used as described herein. For example, an expression cassette can also include nucleotide sequences that encode a signal sequence that directs secretion of an expressed protein from the host cell. Transcription termination signals, enhancers, and other nucleic acid sequences that influence gene expression, can also be included in an expression cassette.

A "recombinant polynucleotide" or a "recombinant polypeptide" is a non-naturally occurring polynucleotide or polypeptide that includes nucleic acid or amino acid sequences, respectively, from more than one source nucleic acid or polypeptide, which source nucleic acid or polypeptide can be a naturally occurring nucleic acid or polypeptide, or can itself have been subjected to mutagenesis or other type of modification. The source polynucleotides or polypeptides from which the different nucleic acid or amino acid sequences are derived are sometimes homologous (*i.e.*, have, or encode a polypeptide that encodes, the same or a similar structure and/or function), and are often from different isolates, serotypes, strains, species, of organism or from different disease states, for example.

The terms "identical" or percent "identity," in the context of two or more nucleic acid or polypeptide sequences, refer to two or more sequences or subsequences that are the same or have a specified percentage of amino acid residues or nucleotides that are the same, when compared and aligned for maximum correspondence, as measured using one of the following sequence comparison algorithms or by visual inspection.

The phrase "substantially identical," in the context of two nucleic acids or polypeptides, refers to two or more sequences or subsequences that have at least 60%, preferably 80%, most preferably 90-95% nucleotide or amino acid residue identity, when compared and aligned for maximum correspondence, as measured using one of the following sequence comparison algorithms or by visual inspection. Preferably, the substantial identity exists over a region of the sequences that is at least about 50 residues in length, more preferably over a region of at least about 100 residues, and most preferably the sequences are substantially identical over at least about 150 residues. In some embodiments, the sequences are substantially identical over the entire length of the coding regions.

For sequence comparison, typically one sequence acts as a reference sequence to which test sequences are compared. When using a sequence comparison algorithm, test and reference sequences are input into a computer, subsequence coordinates are designated, if necessary, and sequence algorithm program parameters are designated. The sequence comparison algorithm then calculates the percent sequence identity for the test sequence(s) relative to the reference sequence, based on the designated program parameters.

Optimal alignment of sequences for comparison can be conducted, e.g., by the local homology algorithm of Smith & Waterman, *Adv. Appl. Math.* 2:482 (1981), by the homology alignment algorithm of Needleman & Wunsch, *J. Mol. Biol.* 48:443 (1970), by the search for similarity method of Pearson & Lipman, *Proc. Nat'l. Acad. Sci. USA* 85:2444 (1988), by computerized implementations of these algorithms (GAP, BESTFIT, FASTA, and TFASTA in the Wisconsin Genetics Software Package, Genetics Computer Group, 575 Science Dr., Madison, WI), or by visual inspection (*see generally* Ausubel *et al., infra*).

One example of an algorithm that is suitable for determining percent sequence identity and sequence similarity is the BLAST algorithm, which is described in Altschul *et al., J. Mol. Biol.* 215:403-410 (1990). Software for performing BLAST analyses is publicly available through the National Center for Biotechnology Information (http://www.ncbi.nlm.nih.gov/). This algorithm involves first identifying high scoring sequence pairs (HSPs) by identifying short words of length W in the query sequence, which either match or satisfy some positive-valued threshold score T when aligned with a word of the same length in a database sequence. T is referred to as the neighborhood word score threshold (Altschul *et al., supra*)*.* These initial neighborhood word hits act as seeds for initiating searches to find longer HSPs containing them. The word hits are then extended in both directions along each sequence for as far as the cumulative alignment score can be increased. Cumulative scores are calculated using, for nucleotide sequences, the parameters M (reward score for a pair of matching residues; always > 0) and N (penalty score for mismatching residues; always < 0). For amino acid sequences, a scoring matrix is used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. The BLASTN program (for nucleotide sequences) uses as defaults a wordlength (W) of 11, an expectation (E) of 10, a cutoff of 100, M=5, N=-4, and a comparison of both strands. For amino acid sequences, the BLASTP program uses as defaults a wordlength (W) of 3, an expectation (E) of 10, and the BLOSUM62 scoring matrix (*see* Henikoff & Henikoff (1989) *Proc. Natl. Acad. Sci. USA* 89:10915).

In addition to calculating percent sequence identity, the BLAST algorithm also performs a statistical analysis of the similarity between two sequences (*see, e.g.,* Karlin & Altschul (1993) *Proc. Nat'l. Acad. Sci. USA* 90:5873-5787). One measure of similarity provided by the BLAST algorithm is the smallest sum probability (P(N)), which provides an indication of the probability by which a match between two nucleotide or amino acid sequences would occur by chance. For example, a nucleic acid is considered similar to a reference sequence if the smallest sum probability in a comparison of the test nucleic acid to the reference nucleic acid is less than about 0.1, more preferably less than about 0.01, and most preferably less than about 0.001.

Another indication that two nucleic acid sequences are substantially identical is that the two molecules hybridize to each other under stringent conditions. The phrase "hybridizing specifically to", refers to the binding, duplexing, or hybridizing of a molecule only to a particular nucleotide sequence under stringent conditions when that sequence is present in a complex mixture (*e.g.*, total cellular) DNA or RNA. "Bind(s) substantially" refers to complementary hybridization between a probe nucleic acid and a target nucleic acid and embraces minor mismatches that can be accommodated by reducing the stringency of the hybridization media to achieve the desired detection of the target polynucleotide sequence.

"Stringent hybridization conditions" and "stringent hybridization wash conditions" in the context of nucleic acid hybridization experiments such as Southern and northern hybridizations are sequence dependent, and are different under different environmental parameters. Longer sequences hybridize specifically at higher temperatures. An extensive guide to the hybridization of nucleic acids is found in Tijssen (1993) *Laboratory Techniques in Biochemistry and Molecular Biology--Hybridization with Nucleic Acid Probes* part I chapter 2 "Overview of principles of hybridization and the strategy of nucleic acid probe assays", Elsevier, New York. Generally, highly stringent hybridization and wash conditions are selected to be about 5° C lower than the thermal melting point (Tₘ) for the specific sequence at a defined ionic strength and pH. Typically, under "stringent conditions" a probe will hybridize to its target subsequence, but to no other sequences.

The Tₘ is the temperature (under defined ionic strength and pH) at which 50% of the target sequence hybridizes to a perfectly matched probe. Very stringent conditions are selected to be equal to the Tₘ for a particular probe. An example of stringent hybridization conditions for hybridization of complementary nucleic acids which have more than 100 complementary residues on a filter in a Southern or northern blot is 50% formamide with 1 mg of heparin at 42°C, with the hybridization being carried out overnight. An example of highly stringent wash conditions is 0.15M NaCl at 72°C for about 15 minutes. An example of stringent wash conditions is a 0.2x SSC wash at 65°C for 15 minutes (*see*, Sambrook, *infra.*, for a description of SSC buffer). Often, a high stringency wash is preceded by a low stringency wash to remove background probe signal. An example medium stringency wash for a duplex of, *e.g.*, more than 100 nucleotides, is 1x SSC at 45°C for 15 minutes. An example low stringency wash for a duplex of, *e.g.*, more than 100 nucleotides, is 4-6x SSC at 40°C for 15 minutes. For short probes (*e.g.*, about 10 to 50 nucleotides), stringent conditions typically involve salt concentrations of less than about 1.0 M Na⁺ ion, typically about 0.01 to 1.0 M Na⁺ ion concentration (or other salts) at pH 7.0 to 8.3, and the temperature is typically at least about 30°C. Stringent conditions can also be achieved with the addition of destabilizing agents such as formamide. In general, a signal to noise ratio of 2x (or higher) than that observed for an unrelated probe in the particular hybridization assay indicates detection of a specific hybridization. Nucleic acids which do not hybridize to each other under stringent conditions are still substantially identical if the polypeptides which they encode are substantially identical. This occurs, *e.g.,* when a copy of a nucleic acid is created using the maximum codon degeneracy permitted by the genetic code.

A further indication that two nucleic acid sequences or polypeptides are substantially identical is that the polypeptide encoded by the first nucleic acid is immunologically cross reactive with, or specifically binds to, the polypeptide encoded by the second nucleic acid. Thus, a polypeptide is typically substantially identical to a second polypeptide, for example, where the two peptides differ only by conservative substitutions.

The phrase "specifically (or selectively) binds to an antibody" or "specifically (or selectively) immunoreactive with", when referring to a protein or peptide, refers to a binding reaction which is determinative of the presence of the protein, or an epitope from the protein, in the presence of a heterogeneous population of proteins and other biologics. Thus, under designated immunoassay conditions, the specified antibodies bind to a particular protein and do not bind in a significant amount to other proteins present in the sample. The antibodies raised against a multivalent antigenic polypeptide will generally bind to the proteins from which one or more of the epitopes were obtained. Specific binding to an antibody under such conditions may require an antibody that is selected for its specificity for a particular protein. A variety of immunoassay formats may be used to select antibodies specifically immunoreactive with a particular protein. For example, solid-phase ELISA immunoassays, Western blots, or immunohistochemistry are routinely used to select monoclonal antibodies specifically immunoreactive with a protein. *See* Harlow and Lane (1988) *Antibodies, A Laboratory Manual,* Cold Spring Harbor Publications, New York "Harlow and Lane"), for a description of immunoassay formats and conditions that can be used to determine specific immunoreactivity. Typically a specific or selective reaction will be at least twice background signal or noise and more typically more than 10 to 100 times background.

"Conservatively modified variations" of a particular polynucleotide sequence refers to those polynucleotides that encode identical or essentially identical amino acid sequences, or where the polynucleotide does not encode an amino acid sequence, to essentially identical sequences. Because of the degeneracy of the genetic code, a large number of functionally identical nucleic acids encode any given polypeptide. For instance, the codons CGU, CGC, CGA, CGG, AGA, and AGG all encode the amino acid arginine. Thus, at every position where an arginine is specified by a codon, the codon can be altered to any of the corresponding codons described without altering the encoded polypeptide. Such nucleic acid variations are "silent variations," which are one species of "conservatively modified variations." Every polynucleotide sequence described herein which encodes a polypeptide also describes every possible silent variation, except where otherwise noted. One of skill will recognize that each codon in a nucleic acid (except AUG, which is ordinarily the only codon for methionine) can be modified to yield a functionally identical molecule by standard techniques. Accordingly, each "silent variation" of a nucleic acid which encodes a polypeptide is implicit in each described sequence.

Furthermore, one of skill will recognize that individual substitutions, deletions or additions which alter, add or delete a single amino acid or a small percentage of amino acids (typically less than 5%, more typically less than 1%) in an encoded sequence are "conservatively modified variations" where the alterations result in the substitution of an amino acid with a chemically similar amino acid. Conservative substitution tables providing functionally similar amino acids are well known in the art. The following five groups each contain amino acids that are conservative substitutions for one another:
Aliphatic: Glycine (G), Alanine (A), Valine (V), Leucine (L), Isoleucine (I);
Aromatic: Phenylalanine (F), Tyrosine (Y), Tryptophan (W);
Sulfur-containing: Methionine (M), Cysteine (C);
Basic: Arginine (R), Lysine (K), Histidine (H);
Acidic: Aspartic acid (D), Glutamic acid (E), Asparagine (N), Glutamine (Q).
*See also,* Creightón (1984) *Proteins,* W.H. Freeman and Company, for additional groupings of amino acids. In addition, individual substitutions, deletions or additions which alter, add or delete a single amino acid or a small percentage of amino acids in an encoded sequence are also "conservatively modified variations".

A "subsequence" refers to a sequence of nucleic acids or amino acids that comprise a part of a longer sequence of nucleic acids or amino acids (*e.g.*, polypeptide) respectively.

### Description of the Preferred Embodiments

The present invention provides methods for obtaining polynucleotide sequences that, either directly or indirectly (*i.e.,* through encoding a polypeptide), can modulate an immune response when present on a genetic vaccine vector. In another embodiment, the invention provides methods for optimizing the transport and presentation of antigens. The optimized immunomodulatory polynucleotides obtained using the methods of the invention are particularly suited for use in conjunction with vaccines, including genetic vaccines. One of the advantages of genetic vaccines is that one can incorporate genes encoding immunomodulatory molecules, such as cytokines, costimulatory molecules, and molecules that improve antigen transport and presentation into the genetic vaccine vectors. This provides opportunities to modulate immune responses that are induced against the antigens expressed by the genetic vaccines.

### A. Creation of Recombinant Libraries

The invention involves creating recombinant libraries of polynucleotides that are then screened to identify those library members that exhibit a desired property. The recombinant libraries can be created using any of various methods.

The substrate nucleic acids used for the recombination can vary depending upon the particular application. For example, where a polynucleotide that encodes a cytokine, chemokine, or other accessory molecule is to be optimized, different forms of nucleic acids that encode all or part of the cytokine, chemokine, or other accessory molecule are subjected to recombination. The methods require at least two variant forms of a starting substrate. The variant forms of candidate substrates can show substantial sequence or secondary structural similarity with each other, but they should also differ in at least two positions. The initial diversity between forms can be the result of natural variation, *e.g.*, the different variant forms (homologs) are obtained from different individuals or strains of an organism (including geographic variants) or constitute related sequences from the same organism (*e.g.*, allelic variations). Alternatively, the initial diversity can be induced, *e.g.,* the second variant form can be generated by error-prone transcription, such as an error-prone PCR or use of a polymerase which lacks proof-reading activity (*see* Liao (1990) *Gene* 88:107-111), of the first variant form, or, by replication of the first form in a mutator strain (mutator host cells are discussed in further detail below). The initial diversity between substrates is greatly augmented in subsequent steps of recursive sequence recombination.

Often, improvements are achieved after one round of recombination and selection. However, recursive sequence recombination can be employed to achieve still further improvements in a desired property. Sequence recombination can be achieved in many different formats and permutations of formats, as described in further detail below. These formats share some common principles. Recursive sequence recombination entails successive cycles of recombination to generate molecular diversity. That is, one creates a family of nucleic acid molecules showing some sequence identity to each other but differing in the presence of mutations. In any given cycle, recombination can occur *in vivo* or *in vitro,* intracellular or extracellular. Furthermore, diversity resulting from recombination can be augmented in any cycle by applying prior methods of mutagenesis (*e.g*., error-prone PCR or cassette mutagenesis) to either the substrates or products for recombination. In some instances, a new or improved property or characteristic can be achieved after only a single cycle of *in vivo* or *in vitro* recombination, as when using different, variant forms of the sequence, as homologs from different individuals or strains of an organism, or related sequences from the same organism, as allelic variations.

In a presently preferred embodiment, the recombinant libraries are prepared using DNA shuffling. The shuffling and screening or selection can be used to "evolve" individual genes, whole plasmids or viruses, multigene clusters, or even whole genomes (Stemmer (1995) *Bio*/*Technology* 13:549-553). Reiterative cycles of recombination and screening/selection can be performed to further evolve the nucleic acids of interest. Such techniques do not require the extensive analysis and computation required by conventional methods for polypeptide engineering. Shuffling allows the recombination of large numbers of mutations in a minimum number of selection cycles, in contrast to traditional, pairwise recombination events. Thus, the sequence recombination techniques described herein provide particular advantages in that they provide recombination between mutations in any or all of these, thereby providing a very fast way of exploring the manner in which different combinations of mutations can affect a desired result. In some instances, however, structural and/or functional information is available which, although not required for sequence recombination, provides opportunities for modification of the technique.

Exemplary formats and examples for sequence recombination, sometimes referred to as DNA shuffling, evolution, or molecular breeding, have been described by the present inventors and co-workers in co-pending applications U.S. Patent Application Serial No. 08/198,431, filed February 17, 1994, Serial No. PCT/US95/02126, filed, February 17, 1995, Serial No. 08/425,684, filed April 18, 1995, Serial No. 08/537,874, filed October 30, 1995, Serial No. 08/564,955, filed November 30, 1995, Serial No. 08/621,859, filed March 25, 1996, Serial No. 08/621,430, filed March 25, 1996, Serial No. PCT/US96/05480, filed April 18, 1996, Serial No. 08/650,400, filed May 20, 1996, Serial No. 08/675,502, filed July 3, 1996, Serial No. 08/721, 824, filed September 27, 1996, Serial No. PCT/LTS97/17300, filed September 26, 1997, and Serial No. PCT/US97/24239, filed December 17, 1997; Stemmer, *Science* 270:1510 (1995); Stemmer *et al., Gene* 164:49-53 (1995); Stemmer, *Bio*/*Technology* 13:549-553 (1995); Stemmer, *Proc. Natl. Acad. Sci. U.S.A.* 91:10747-10751 (1994); Stemmer, *Nature* 370:389-391 (1994); Crameri *et al., Nature Medicine* 2(1):1-3 (1996); Crameri *et al., Nature Biotechnology* 14:315-319 (1996), each of which is incorporated by reference in its entirety for all purposes.

Other methods for obtaining recombinant polynucleotides and/or for obtaining diversity in nucleic acids used as the substrates for shuffling include, for example, homologous recombination (PCT/US98/05223; Publ. No. WO98/42727); oligonucleotide-directed mutagenesis (for review *see,* Smith, *Ann. Rev. Genet.* 19: 423-462 (1985); Botstein and Shortle, *Science* 229: 1193-1201 (1985); Carter, *Biochem. J.* 237: 1-7 (1986); Kunkel, "The efficiency of oligonucleotide directed mutagenesis" *in Nucleic acids & Molecular Biology,* Eckstein and Lilley, eds., Springer Verlag, Berlin (1987)). Included among these methods are oligonucleotide-directed mutagenesis (Zoller and Smith, *Nucl. Acids Res.* 10: 6487-6500 (1982), *Methods in Enzymol.* 100: 468-500 (1983), and *Methods in Enzymol.* 154: 329-350 (1987)) phosphothioate-modified DNA mutagenesis (Taylor *et al., Nucl. Acids Res.* 13: 8749-8764 (1985); Taylor *et al., Nucl. Acids Res.* 13: 8765-8787 (1985); Nakamaye and Eckstein, *Nucl. Acids Res.* 14: 9679-9698 (1986); Sayers *et al., Nucl. Acids Res.* 16: 791-802 (1988); Sayers *et al., Nucl. Acids Res.* 16: 803-814 (1988)), mutagenesis using uracil-containing templates (Kunkel, *Proc. Nat'l. Acad. Sci. USA* 82: 488-492 (1985) and Kunkel *et al., Methods in Enzymol.* 154: 367-382)); mutagenesis using gapped duplex DNA (Kramer *et al., Nucl. Acids Res.* 12: 9441-9456 (1984); Kramer and Fritz, *Methods in Enzymol.* 154: 350-367 (1987); Kramer *et al., Nucl. Acids Res.* 16: 7207 (1988)); and Fritz *et al., Nucl. Acids Res.* 16: 6987-6999 (1988)). Additional suitable methods include point mismatch repair (Kramer *et al., Cell* 38: 879-887 (1984)), mutagenesis using repair-deficient host strains (Carter *et al., Nucl. Acids Res.* 13: 4431-4443 (1985); Carter, *Methods in Enzymol.* 154: 382-403 (1987)), deletion mutagenesis (Eghtedarzadeh and Henikoff, *Nucl. Acids Res.* 14: 5115 (1986)), restriction-selection and restriction-purification (Wells *et al., Phil. Trans. R. Soc. Lond.* A 317: 415-423 (1986)), mutagenesis by total gene synthesis (Nambiar *et al., Science* 223: 1299-1301 (1984); Sakamar and Khorana, *Nucl. Acids Res.* 14: 6361-6372 (1988); Wells *et al., Gene* 34: 315-323 (1985); and Grundström *et al., Nucl. Acids Res.* 13: 3305-3316 (1985). Kits for mutagenesis are commercially available (*e.g.,* Bio-Rad, Amersham International, Anglian Biotechnology).

### B. Screening Methods

A recombination cycle is usually followed by at least one cycle of screening or selection for molecules having a desired property or characteristic. If a recombination cycle is performed *in vitro,* the products of recombination, *i.e*., recombinant segments, are sometimes introduced into cells before the screening step. Recombinant segments can also be linked to an appropriate vector or other regulatory sequences before screening. Alternatively, products of recombination generated *in vitro* are sometimes packaged as viruses before screening. If recombination is performed *in vivo,* recombination products can sometimes be screened in the cells in which recombination occurred. In other applications, recombinant segments are extracted from the cells, and optionally packaged as viruses, before screening.

The nature of screening or selection depends on what property or characteristic is to be acquired or the property or characteristic for which improvement is sought, and many examples are discussed below. It is not usually necessary to understand the molecular basis by which particular products of recombination (recombinant segments) have acquired new or improved properties or characteristics relative to the starting substrates. For example, a genetic vaccine vector can have many component sequences each having a different intended role (*e.g.*, coding sequence, regulatory sequences, targeting sequences, stability-conferring sequences, immunomodulatory sequences, sequences affecting antigen presentation, and sequences affecting integration). Each of these component sequences can be varied and recombined simultaneously. Screening/selection can then be performed, for example, for recombinant segments that have increased episomal maintenance in a target cell without the need to attribute such improvement to any of the individual component sequences of the vector.

Depending on the particular screening protocol used for a desired property, initial round(s) of screening can sometimes be performed in bacterial cells due to high transfection efficiencies and ease of culture. Later rounds, and other types of screening which are not amenable to screening in bacterial cells, are performed in mammalian cells to optimize recombinant segments for use in an environment close to that of their intended use. Final rounds of screening can be performed in the precise cell type of intended use (*e.g.,* a human antigen-presenting cell). In some instances, this cell can be obtained from a patient to be treated with a view, for example, to minimizing problems of immunogenicity in this patient.

The screening or selection step identifies a subpopulation of recombinant segments that have evolved toward acquisition of a new or improved desired property or properties useful in genetic vaccination. Depending on the screen, the recombinant segments can be identified as components of cells, components of viruses or in free form. More than one round of screening or selection can be performed after each round of recombination.

If further improvement in a property is desired, at least one and usually a collection of recombinant segments surviving a first round of screening/selection are subject to a further round of recombination. These recombinant segments can be recombined with each other or with exogenous segments representing the original substrates or further variants thereof. Again, recombination can proceed *in vitro* or *in vivo.* If the previous screening step identifies desired recombinant segments as components of cells, the components can be subjected to further recombination *in vivo,* or can be subjected to further recombination *in vitro,* or can be isolated before performing a round of *in vitro* recombination. Conversely, if the previous screening step identifies desired recombinant segments in naked form or as components of viruses, these segments can be introduced into cells to perform a round of *in vivo* recombination. The second round of recombination, irrespective how performed, generates further recombinant segments which encompass additional diversity than is present in recombinant segments resulting from previous rounds.

The second round of recombination can be followed by a further round of screening/selection according to the principles discussed above for the first round. The stringency of screening/selection can be increased between rounds. Also, the nature of the screen and the property being screened for can vary between rounds if improvement in more than one property is desired or if acquiring more than one new property is desired. Additional rounds of recombination and screening can then be performed until the recombinant segments have sufficiently evolved to acquire the desired new or improved property or function.

Various screening methods for particular applications are described herein. In several instances, screening involves expressing the recombinant peptides or polypeptides encoded by the recombinant polynucleotides of the library as fusions with a protein that is displayed on the surface of a replicable genetic package. For example, phage display can be used. *See, e.g,* Cwirla *et al., Proc. Natl. Acad. Sci.* USA 87: 6378-6382 (1990); Devlin *et al., Science* 249: 404-406 (1990), Scott & Smith, *Science* 249: 386-388 (1990); Ladner *et al.,* US 5,571,698. Other replicable genetic packages include, for example, bacteria, eukaryotic viruses, yeast, and spores.

The genetic packages most frequently used for display libraries are bacteriophage, particularly filamentous phage, and especially phage M13, Fd and F1. Most work has involved inserting libraries encoding polypeptides to be displayed into either gIII or gVIII of these phage forming a fusion protein. *See, e.g.,* Dower, WO 91/19818; Devlin, W 91/18989; MacCafferty, WO 92/01047 (gene III); Huse, W0 92/06204; Kang, WO 92/18619 (gene VIII). Such a fusion protein comprises a signal sequence, usually but not necessarily, from the phage coat protein, a polypeptide to be displayed and either the gene III or gene VIII protein or a fragment thereof. Exogenous coding sequences are often inserted at or near the N-terminus of gene III or gene VIII although other insertion sites are possible.

Eukaryotic viruses can be used to display polypeptides in an analogous manner. For example, display of human heregulin fused to gp70 of Moloney murine leukemia virus has been reported by Han *et al., Proc. Natl. Acad. Sci. USA* 92: 9747-9751 (1995). Spores can also be used as replicable genetic packages. In this case, polypeptides are displayed from the outer surface of the spore. For example, spores from *B. subtilis* have been reported to be suitable. Sequences of coat proteins of these spores are provided by Donovan *et al., J. Mol. Biol.* 196, 1-10 (1987). Cells can also be used as replicable genetic packages. Polypeptides to be displayed are inserted into a gene encoding a cell protein that is expressed on the cells surface. Bacterial cells including *Salmonella typhimurium, Bacillus subtilis, Pseudomonas aeruginosa, Vibrio cholerae, Klebsiella pneumonia, Neisseria gonorrhoeae, Neisseria meningitidis, Bacteroides nodosus, Moraxella bovis,* and especially *Escherichia coli* are preferred. Details of outer surface proteins are discussed by Ladner et al., US 5,571,698 and references cited therein. For example, the *lam*B protein of *E. coli* is suitable.

A basic concept of display methods that use phage or other replicable genetic package is the establishment of a physical association between DNA encoding a polypeptide to be screened and the polypeptide. This physical association is provided by the replicable genetic package, which displays a polypeptide as part of a capsid enclosing the genome of the phage or other package, wherein the polypeptide is encoded by the genome. The establishment of a physical association between polypeptides and their genetic material allows simultaneous mass screening of very large numbers of phage bearing different polypeptides. Phage displaying a polypeptide with affinity to a target, *e.g.*, a receptor, bind to the target and these phage are enriched by affinity screening to the target. The identity of polypeptides displayed from these phage can be determined from their respective genomes. Using these methods a polypeptide identified as having a binding affinity for a desired target can then be synthesized in bulk by conventional means, or the polynucleotide that encodes the peptide or polypeptide can be used as part of a genetic vaccine.

### C. Evolution of Improved Immunomodulatory Sequences

Cytokines can dramatically influence macrophage activation and T_{H}1/T_{H}2 cell differentiation, and thereby the outcome of infectious diseases. In addition, recent studies strongly suggest that DNA itself can act as adjuvant by activating the cells of the immune system. Specifically, unmethylated CpG-rich DNA sequences were shown to enhance T_{H}1 cell differentiation, activate cytokine synthesis by monocytes and induce proliferation of B lymphocytes. The invention thus provides methods for enhancing the immunomodulatory properties of genetic vaccines (a) by evolving the stimulatory properties of DNA itself and (b) by evolving genes encoding cytokines and related molecules that are involved in immune system regulation. These genes are then used in genetic vaccine vectors.

Of particular interest are IFN-α and IL-12, which skew immune responses towards a T helper 1 (T_{H}1) cell phenotype and, thereby, improve the host's capacity to counteract pathogen invasions. Also provided are methods of obtaining improved immunomodulatory nucleic acids that are capable of inhibiting or enhancing activation, differentiation, or anergy of antigen-specific T cells. Because of the limited information about the structures and mechanisms that regulate these events, molecular breeding techniques of the invention provide much faster solutions than rational design.

The methods of the invention typically involve the use of DNA shuffling or other methods to create a library of recombinant polynucleotides. The library is then screened to identify recombinant polynucleotides in the library, when included in a genetic vaccine vector or administered in conjunction with a genetic vaccine, are capable of enhancing or otherwise altering an immune response induced by the vector. The screening step, in some embodiments, can involve introducing a genetic vaccine vector that includes the recombinant polynucleotides into mammalian cells and determining whether the cells, or culture medium obtained by growing the cells, is capable of modulating an immune response.

Optimized recombinant vector modules obtained through polynucleotide recombination are useful not only as components of genetic vaccine vectors, but also for production of polypeptides, *e.g.*, modified cytokines and the like, that can be administered to a mammal to enhance or shift an immune response. Polynucleotide sequences obtained using the DNA shuffling methods of the invention can be used as a component of a genetic vaccine, or can be used for production of cytokines and other immunomodulatory polypeptides that are themselves used as therapeutic or prophylactic reagents. If desired, the sequence of the optimized immunomodulatory polypeptide-encoding polynucleotides can be determined and the deduced amino acid sequence used to produce polypeptides using methods known to those of skill in the art.

### 1. Immunostimulatory DNA sequences

The invention provides methods of obtaining polynucleotides that are immunostimulatory when introduced into a mammal. Oligonucleotides that contain hexamers with a central CpG flanked by two 5' purines (GpA or ApA) and two 3' pyrimidines (TpC or TpT) efficiently induce cytokine synthesis and B cell proliferation (Krieg *et al.* (1995) *Nature* 374: 546; Klinman *et al.* (1996) *Proc. Nat'l. Acad. Sci. USA* 93: 2879; Pisetsky (1996) *Immunity* 5: 303-10) *in vitro* and act as adjuvants *in vivo.* Genetic vaccine vectors in which immunostimulatory sequence- (ISS) containing oligos are inserted have increased capacity to enhance antigen-specific antibody responses after DNA vaccination. The minimal length of an ISS oligonucleotide for functional activity *in vitro* is eight (Klinman *et al., supra.*). Twenty-mers with three CG motifs were found to be significantly more efficient in inducing cytokine synthesis than a 15-mer with two CG motifs (*Id.*). GGGG tetrads have been suggested to be involved in binding of DNA to cell surfaces (macrophages express receptors. for example scavenger receptors, that bind DNA) (Pisetsky *et al., supra*.).

According to the invention, a library is generated by subjecting to recombination random DNA (*e.g.*, fragments of human, murine, or other genomic DNA), oligonucleotides that contain known ISS, poly A, C, G or T sequences, or combinations thereof. The DNA, which includes at least first and second forms which differ from each other in two or more nucleotides, are recombined to produce a library ofrecombinant polynucleotides.

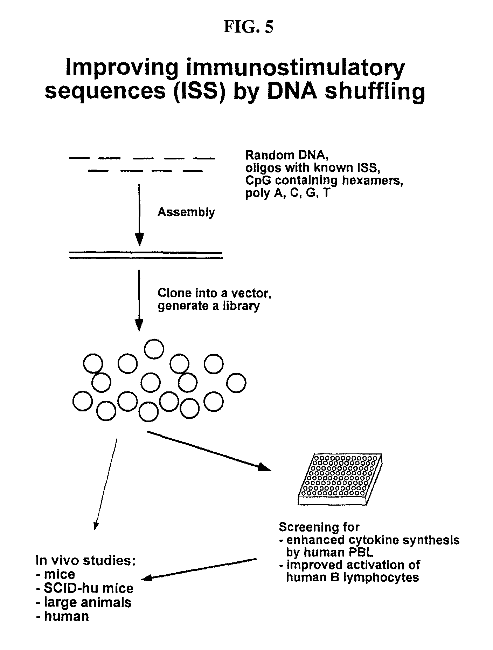

The library is then screened to identify those recombinant polynucleotides that exhibit immunostimulatory properties. For example, the library can be screened for induction cytokine production *in vitro* upon introduction of the library into an appropriate cell type. A diagram of this procedure is shown in Figure 5. Among the cytokines that can be used as an indicator of immunostimulatory activity are, for example, IL-2, IL-4, IL-5, IL-6, IL-10, IL-12, IL-13, IL-15, and IFN-γ. One can also test for changes in ratios of IL-4/IFN-γ, IL-4/IL-2, IL-5/IFN-γ, IL-5/IL-2, IL-13/IFN-γ, IL-13/IL-2. An alternative screening method is the determination of the ability to induce proliferation of cells involved in immune responses, such as B cells, T cells, monocytes/macrophages, total PBL, and the like. Other screens include detecting induction of APC activation based on changes in expression levels of surface antigens, such as B7-1 (CD80), B7-2 (CD86), MHC class I and II, and CD14.

Other useful screens include identifying recombinant polynucleotides that induce T cell proliferation. Because ISS sequences induce B cell activation, and because of several homologies between surface antigens expressed by T cells and B cells, polynucleotides can be obtained that have stimulatory activities on T cells.

Libraries of recombinant polynucleotides can also be screened for improved CTL and antibody responses *in vivo* and for improved protection from infection, cancer, allergy or autoimmunity. Recombinant polynucleotides that exhibit the desired property can be recovered from the cell and, if further improvement is desired, the shuffling and screening can be repeated. Optimized ISS sequences can used as an adjuvant separately from an actual vaccine, or the DNA sequence of interest can be fused to a genetic vaccine vector.

### 2. Cytokines, chemokines, and accessory molecules

The invention also provides methods for obtaining optimized cytokines, cytokine antagonists, chemokines, and other accessory molecules that direct, inhibit, or enhance immune responses. For example, the methods of the invention can be used to obtain genetic vaccines and other reagents (e.g., optimized cytokines, and the like) that, when administered to a mammal, improve or alter an immune response. These optimized immunomodulators are useful for treating infectious diseases, as well as other conditions such as inflammatory disorders, in an antigen non-specific manner.

For example, the methods of the invention can be used to develop optimized immunomodulatory molecules for treating allergies. The optimized immunomodulatory molecules can be used alone or in conjunction with antigen-specific genetic vaccines to prevent or treat allergy. Four basic mechanisms are available by which one can achieve specific immunotherapy of allergy. First, one can administer a reagent that causes a decrease in allergen-specific T_{H}2 cells. Second, a reagent can be administered that causes an increase in allergen-specific T_{H}1 cells. Third, one can direct an increase in suppressive CD8⁺ T cells. Finally, allergy can be treated by inducing anergy of allergen-specific T cells. In this Example, cytokines are optimized using the methods of the invention to obtain reagents that are effective in achieving one or more of these immunotherapeutic goals. The methods of the invention are used to obtain anti-allergic cytokines that have one or more properties such as improved specific activity, improved secretion after introduction into target cells, are effective at a lower dose than natural cytokines, and fewer side effects. Targets of particular interest include interferon-α/γ, IL-10, IL-12, and antagonists of IL-4 and IL-13.

The optimized immunomodulators, or optimized recombinant polynucleotides that encode the immunomodulators, can be administered alone, or in combination with other accessory molecules. Inclusion of optimal concentrations of the appropriate molecules can enhance a desired immune response, and/or direct the induction or repression of a particular type of immune response. The polynucleotides that encode the optimized molecules can be included in a genetic vaccine vector, or the optimized molecules encoded by the genes can be administered as polypeptides.

In the methods of the invention, a library of recombinant polynucleotides that encode immunomodulators is created by subjecting substrate nucleic acids to a recombination protocol, such as DNA shuffling or other method known to those of skill in the art. The substrate nucleic acids are typically two or more forms of a nucleic acid that encodes an immunomodulator of interest.

Cytokines are among the immunomodulators that can be improved using the methods of the invention. Cytokine synthesis profiles play a crucial role in the capacity of the host to counteract viral, bacterial and parasitic infections, and cytokines can dramatically influence the efficacy of genetic vaccines and the outcome of infectious diseases. Several cytokines, for example IL-1, IL-2, IL-3, IL-4, IL-5, IL-6, IL-7, IL-8, IL-9, IL-10, IL-11, IL-12, IL-13, IL-14, IL-15, IL-16, IL-17, IL-18, G-CSF, GM-CSF, IFN-α, IFN-γ, TGF-β, TNF-α, TNF-β, IL-20 (MDA-7), and fit-3 ligand have been shown stimulate immune responses *in vitro* or *in vivo.* Immune functions that can be enhanced using appropriate cytokines include, for example, B cell proliferation, Ig synthesis, Ig isotype switching, T cell proliferation and cytokine synthesis, differentiation of T_{H}1 and T_{H}2 cells, activation and proliferation of CTLs, activation and cytokine production by monocytes/macrophages/dendritic cells, and differentiation of dendritic cells from monocytes/macrophages.

In some embodiments, the invention provides methods of obtaining optimized immunomodulators that can direct an immune response towards a T_{H}1 or a T_{H}2 response. The ability to influence the direction of immune responses in this manner is of great importance in development of genetic vaccines. Altering the type of T_{H} response can fundamentally change the outcome of an infectious disease. A high frequency of T_{H}1 cells generally protects from lethal infections with intracellular pathogens, whereas a dominant T_{H}2 phenotype often results in disseminated, chronic infections. For example, in human, the T_{H}1 phenotype is present in the tuberculoid (resistant) form of leprosy, while the T_{H}2 phenotype is found in lepromatous, multibacillary (susceptible) lesions (Yamamura *et al.* (1991) *Science* 254: 277). Late-stage AIDS patients have the T_{H}2 phenotype. Studies in family members indicate that survival from meningococcal septicemia depends on the cytokine synthesis profile of PBL, with high IL-10 synthesis being associated with a high risk of lethal outcome and high TNF-α being associated with a low risk. Similar examples are found in mice. For example, BALB/c mice are susceptible to *Leishmania major* infection; these mice develop a disseminated fatal disease with a T_{H}2 phenotype. Treatment with anti-IL-4 monoclonal antibodies or with IL-12 induces a T_{H}1 response, resulting in healing. Anti-interferon-γ monoclonal antibodies exacerbate the disease. For some applications, it is preferable to direct an immune response in the direction of a T_{H}2 response. For example, where increased mucosal immunity is desired, including protective immunity, enhancing the T_{H}2 response can lead to increased antibody production, particularly IgA.

T helper (T_{H}) cells are probably the most important regulators of the immune system. T_{H} cells are divided into two subsets, based on their cytokine synthesis pattern (Mosmann and Coffman (1989) *Adv. Immunol.* 46: 111). T_{H}1 cells produce high levels of the cytokines IL-2 and IFN-γ and no or minimal levels of IL-4, IL-5 and IL-13. In contrast, T_{H}2 cells produce high levels of IL-4, IL-5 and IL-13, and IL-2 and IFN-γ production is minimal or absent. T_{H}1 cells activate macrophages, dendritic cells and augment the cytolytic activity of CD8⁺ cytotoxic T lymphocytes and natural killer (NK) cells (Paul and Seder (1994) *Cell* 76: 241), whereas T_{H}2 cells provide efficient help for B cells and also mediate allergic responses due to the capacity of T_{H}2 cells to induce IgE isotype switching and differentiation of B cells into IgE secreting cells (Punnonen *et al.* (1993) *Proc. Nat'l. Acad. Sci. USA* 90: 3730).

The screening methods for improved cytokines, chemokines, and other accessory molecules are generally based on identification of modified molecules that exhibit improved specific activity on target cells that are sensitive to the respective cytokine, chemokine, or other accessory molecules. A library of recombinant cytokine, chemokine, or accessory molecule nucleic acids can be expressed on phage or as purified protein and tested using *in vitro* cell culture assays, for example. Importantly, when analyzing the recombinant nucleic acids as components of DNA vaccines, one can identify the most optimal DNA sequences (in addition to the functions of the protein products) in terms of their immunostimulatory properties, transfection efficiency, and their capacity to improve the stabilities of the vectors. The identified optimized recombinant nucleic acids can then be subjected to new rounds of shuffling and selection.

In one embodiment of the invention, cytokines are evolved that direct differentiation of T_{H}1 cells. Because of their capacities to skew immune responses towards a T_{H}1 phenotype, the genes encoding interferon-α (IFN-α) and interleukin-12 (IL-12) are preferred substrates for recombination and selection in order to obtain maximal specific activity and capacity to act as adjuvants in genetic vaccinations. IFN-α is a particularly preferred target for optimization using the methods of the invention because of its effects on the immune system, tumor cells growth and viral replication. Due to these activities, IFN-α was the first cytokine to be used in clinical practice. Today, IFN-α is used for a wide variety of applications, including several types of cancers and viral diseases. IFN-α also efficiently directs differentiation of human T cells into T_{H}1 phenotype (Parronchi *et al.* (1992) *J. Immunol.* 149: 2977). However, it has not been thoroughly investigated in vaccination models, because, in contrast to human systems, it does not affect T_{H}1 differentiation in mice. The species difference was recently explained by data indicating that, like IL-12, IFN-α induces STAT4 activation in human cells but not in murine cells, and STAT4 has been shown to be required in IL-12 mediated T_{H}1 differentiation (Thierfelder *et al.* (1996) *Nature* 382: 171).

Family DNA shuffling is a preferred method for optimizing IFN-α, using as substrates the mammalian IFN-α genes, which are 85% - 97% homologous. Greater than 10²⁶ distinct recombinants can be generated from the natural diversity in these genes. To allow rapid parallel analysis of recombinant interferons, one can employ high throughput methods for their expression and biological assay as fusion proteins on bacteriophage. Recombinants with improved potency and selectivity profiles are being selectively bred for improved activity. Variants which demonstrate improved binding to IFN-α receptors can be selected for further analysis using a screen for mutants with optimal capacity to direct T_{H}1 differentiation. More specifically, the capacities of IFN-α mutants to induce IL-2 and IFN-γ production in *in vitro* human T lymphocyte cultures can be studied by cytokine-specific ELISA and cytoplasmic cytokine staining and flow cytometry.

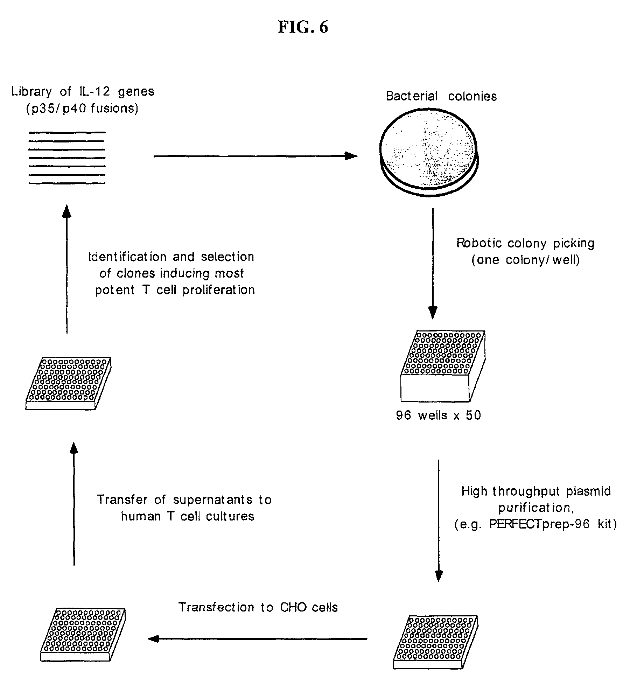

IL-12 is perhaps the most potent cytokine that directs T_{H}1 responses, and it has also been shown to act as an adjuvant and enhance T_{H}1 responses following genetic vaccinations (Kim *et al.* (1997) *J. Immunol.* 158: 816). IL-12 is both structurally and functionally a unique cytokine. It is the only heterodimeric cytokine known to date, composed of a 35 kD light chain (p35) and a 40 kD heavy chain (p40) (Kobayashi *et al* (1989) *J*. *Exp. Med.* 170: 827; Stern *et al.* (1990) *Proc. Nat'l. Acad. Sci. USA* 87: 6808). Recently Lieschke *et al.* ((1997) *Nature Biotech.* 15: 35) demonstrated that a fusion between p35 and p40 genes results in a single gene that has activity comparable to that of the two genes expressed separately. These data indicate that it is possible to shuffle IL-12 genes as one entity, which is beneficial in designing the shuffling protocol. Because of its T cell growth promoting activities, one can use normal human peripheral blood T cells in the selection of the most active IL-12 genes, enabling direct selection of IL-12 mutants with the most potent activities on human T cells. IL-12 mutants can be expressed in CHO cells, for example, and the ability of the supernatants to induce T cell proliferation determined (Figure 6). The concentrations of IL-12 in the supernatants can be normalized based on a specific ELISA that detects a tag fused to the shuffled IL-12 molecules.

Incorporation of evolved IFN-α and/or IL-12 genes into genetic vaccine vectors is expected to be safe. The safety of IFN-α has been demonstrated in numerous clinical studies and in everyday hospital practice. A Phase II trial of IL-12 in the treatment of patients with renal cell cancer resulted in several unexpected adverse effects (Tahara *et al.* (1995) *Human Gene Therapy* 6: 1607). However, IL-12 gene as a component of genetic vaccines aims at high local expression levels, whereas the levels observed in circulation are minimal compared to those observed after systemic bolus injections. In addition, some of the adverse effects of systemic IL-12 treatments are likely to be related to its unusually long half-life (up to 48 hours in monkeys). DNA shuffling may allow selection for a shorter half-life, thereby reducing the toxicity even after high bolus doses.

In other cases, genetic vaccines that can induce T_{H}2 responses are preferred, especially when improved antibody production is desired. As an example, IL-4 has been shown to direct differentiation of T_{H}2 cells (which produce high levels of IL-4, IL-5 and IL-13, and mediate allergic immune responses). Immune responses that are skewed towards T_{H}2 phenotype are preferred when genetic vaccines are used to immunize against autoimmune diseases prophylactically. T_{H}1 responses are also preferred when the vaccines are used to treat and modulate existing autoimmune responses, because autoreactive T cells are generally of T_{H}1 phenotype (Liblau *et al.* (1995) *Immunol. Today* 16:34-38). IL-4 is also the most potent cytokine in induction of IgE synthesis; IL-4 deficient mice are unable to produce IgE. Asthma and allergies are associated with an increased frequency of IL-4 producing cells, and are genetically linked to the locus encoding IL-4, which is on chromosome 5 (in close proximity to genes encoding IL-3, IL-5, IL-9, IL-13 and GM-CSF). IL-4, which is produced by activated T cells, basophils and mast cells, is a protein that has 153 amino acids and two potential N-glycosylation sites. Human IL-4 is only approximately 50% identical to mouse IL-4, and IL-4 activity is species-specific. In human, IL-13 has activities similar to those of IL-4, but IL-13 is less potent than IL-4 in inducing IgE synthesis. IL-4 is the only cytokine known to direct T_{H}2 differentiation.

Improved IL-2 agonists are also useful in directing T_{H}2 cell differentiation, whereas improved IL-4 antagonists can direct T_{H}1 cell differentiation. Improved IL-4 agonists and antagonists can be generated by shuffling of IL-4 or soluble IL-4 receptor. The IL-4 receptor consists of an IL-4R α-chain (140 kD high-affinity binding unit) and an IL-2R γ-chain (these cytokine receptors share a common γ-chain). The IL-4R α-chain is shared by IL-4 and IL-13 receptor complexes. Both IL-4 and IL-13 induce phosphorylation of the IL-4R α-chain, but expression of IL-4R α-chain alone on transfectants is not sufficient to provide a functional IL-4R. Soluble IL-4 receptor currently in clinical trials for the treatment of allergies. Using the DNA shuffling methods of the invention, one can evolve a soluble IL-4 receptor that has improved affinity for IL-4. Such receptors are useful for the treatment of asthma and other T_{H}2 cell mediated diseases, such as severe allergies. The shuffling reactions can take advantage of natural diversity present in cDNA libraries from activated T cells from human and other primates. In a typical embodiment, a shuffled IL-4R α-chain library is expressed on a phage, and mutants that bind to IL-4 with improved affinity are identified. The biological activity of the selected mutants is then assayed using cell-based assays.

IL-2 and IL-15 are also of particular interest for use in genetic vaccines. IL-2 acts as a growth factor for activated B and T cells, and it also modulates the functions of NK-cells. IL-2 is predominantly produced by T_{H}1-like T cell clones, and, therefore, it is considered mainly to function in delayed type hypersensitivity reactions. However, IL-2 also has potent, direct effects on proliferation and Ig-synthesis by B cells. The complex immunoregulatory properties of IL-2 are reflected in the phenotype of IL-2 deficient mice, which have high mortality at young age and multiple defects in their immune functions including spontaneous development of inflammatory bowel disease. IL-15 is a more recently identified cytokine produced by multiple cell types. IL-15 shares several, but not all, activities with IL-2. Both IL-2 and IL-15 induce B cell growth and differentiation. However, assuming that IL-15 production in IL-2 deficient mice is normal, it is clear that IL-15 cannot substitute for the function of IL-2 *in vivo,* since these mice have multiple immunodeficiencies. IL-2 has been shown to synergistically enhance IL-10-induced human Ig production in the presence of anti-CD40 mAbs, but it antagonized the effects of IL-4. IL-2 also enhances IL-4-dependent IgE synthesis by purified B cells. On the other hand, IL-2 was shown to inhibit IL-4-dependent murine IgG1 and IgE synthesis both *in vitro* and *in vivo.* Similarly, IL-2 inhibited IL-4-dependent human IgE synthesis by unfractionated human PBMC, but the effects were less significant than those of IFN-α or IFN-γ. Due to their capacities to activate both B and T cells, IL-2 and IL-15 are useful in vaccinations. In fact, IL-2, as protein and as a component of genetic vaccines, has been shown to improve the efficacy of the vaccinations. Improving the specific activity and/or expression levels/kinetics of IL-2 and IL-15 through use of the DNA shuffling methods of the invention increases the advantageous effects compared to wild-type IL-2 and IL-15.

Another cytokine of particular interest for optimization and use in genetic vaccines according to the methods of the invention is interleukin-6. IL-6 is a monocyte-derived cytokine that was originally described as a B cell differentiation factor or B cell stimulatory factor-2 because of its ability to enhance Ig levels secreted by activated B cells. IL-6 has also been shown to enhance IL-4-induced IgE synthesis. It has also been suggested that IL-6 is an obligatory factor for human IgE synthesis, because neutralizing anti-IL-6 mAbs completely blocked IL-4-induced IgE synthesis. IL-6 deficient mice have impaired capacity to produce IgA. Because of its potent activities on the differentiation ofB cells, IL-6 can enhance the levels of specific antibodies produced following vaccination. It is particularly useful as a component of DNA vaccines because high local concentrations can be achieved, thereby providing the most potent effects on the cells adjacent to the transfected cells expressing the immunogenic antigen. IL-6 with improved specific activity and/or with improved expression levels, obtained by DNA shuffling, will have more beneficial effects than the wild-type IL-6.

Interleukin-8 is another example of a cytokine that, when modified according to the methods of the invention, is useful in genetic vaccines. IL-8 was originally identified as a monocyte-derived neutrophil chemotactic and activating factor. Subsequently, IL-8 was also shown to be chemotactic for T cells and to activate basophils resulting in enhanced histamine and leukotriene release from these cells. Furthermore, IL-8 inhibits adhesion of neutrophils to cytokine-activated endothelial cell monolayers, and it protects these cells from neutrophil-mediated damage. Therefore, endothelial cell derived IL-8 was suggested to attenuate inflammatory events occurring in the proximity of blood vessel walls. IL-8 also modulates immunoglobulin production, and inhibits IL-4-induced IgG4 and IgE synthesis by both unfractionated human PBMC and purified B cells *in vitro.* This inhibitory effect was independent of IFN-α, IFN-γ or prostaglandin E2. In addition, IL-8 inhibited spontaneous IgE synthesis by PBMC derived from atopic patients. Due to its capacity to attract inflammatory cells, IL-8, like other chemotactic agents, is useful in potentiating the functional properties of vaccines, including DNA vaccines (acting as an adjuvant). The beneficial effects of IL-8 can be improved by using the DNA shuffling methods of the invention to obtain IL-8 with improved specific activity and/or with improved expression in target cells.

Interleukin-5, and antagonists thereof, can also be optimized using the methods of the invention for use in genetic vaccines. IL-5 is primarily produced by T_{H}2-type T cells and appears to play an important role in the pathogenesis of allergic disorders because of its ability to induce eosinophilia. IL-5 acts as an eosinophil differentiation and survival factor in both mouse and man. Blocking IL-5 activity by use of neutralizing monoclonal antibodies strongly inhibits pulmonary eosinophilia and hyperactivity in mouse models, and IL-5 deficient mice do not develop eosinophilia. These data also suggest that IL-5 antagonists may have therapeutic potential in the treatment of allergic eosinophilia.

IL-5 has also been shown to enhance both proliferation of, and Ig synthesis by, activated mouse and human B cells. However, other studies suggested that IL-5 has no effect on proliferation of human B cells, whereas it activated eosinophils. IL-5 apparently is not crucial for maturation or differentiation of conventional B cells, because antibody responses in IL-5 deficient mice are normal. However, these mice have a developmental defect in their CD5⁺ B cells indicating that IL-5 is required for normal differentiation of this B cell subset in mice. At suboptimal concentrations of IL-4, IL-5 was shown to enhance IgE synthesis by human B cells *in vitro.* Furthermore, a recent study suggested that the effects of IL-5 on human B cells depend on the mode of B cell stimulation. IL-5 significantly enhanced IgM synthesis by B cells stimulated with *Moraxella catarrhalis.* In addition, IL-5 synergized with suboptimal concentrations of IL-2, but had no effect on Ig synthesis by SAC-activated B cells. Activated human B cells also expressed IL-5 mRNA suggesting that IL-5 may also regulate B cell function, including IgE synthesis, by autocrine mechanisms.

The invention provides methods of evolving an IL-5 antagonist that efficiently binds to and neutralizes IL-5 or its receptor. These antagonists are useful as a component of vaccines used for prophylaxis and treatment of allergies. Nucleic acids encoding IL-5, for example, from human and other mammalian species, are shuffled and screened for binding to immobilized IL-5R for the initial screening. Polypeptides that exhibit the desired effect in the initial screening assays can then be screened for the highest biological activity using assays such as inhibition of growth of IL-5 dependent cells lines cultured in the presence of recombinant wild-type IL-5. Alternatively, shuffled IL-5R α-chains are screened for improved binding to IL-5.

Tumor necrosis factors (α and β) and their receptors are also suitable targets for modification and use in genetic vaccines. TNF-α, which was originally described as cachectin because of its ability to cause necrosis of tumors, is a 17 kDa protein that is produced in low quantities by almost all cells in the human body following activation. TNF-α acts as an endogenous pyrogen and induces the synthesis of several proinflammatory cytokines, stimulates the production of acute phase proteins, and induces proliferation of fibroblasts. TNF-α plays a major role in the pathogenesis of endotoxin shock. A membrane-bound form of TNF-α (mTNF-α), which is involved in interactions between Band T-cells, is rapidly upregulated within four hours of T cell activation. mTNF-α plays a role in the polyclonal B cell activation observed in patients infected with HIV. Monoclonal antibodies specific for mTNF-α or the p55 TNF-α receptor strongly inhibit IgE synthesis induced by activated CD4⁺ T cell clones or their membranes. Mice deficient for p55 TNF-αR are resistant to endotoxic shock, and soluble TNF-aR prevents autoimmune diabetes mellitus in NOD mice. Phase III trials using sTNF-αR in the treatment of rheumatoid arthritis are in progress, after promising results obtained in the phase II trials.

The methods of the invention can be used to, for example, evolve a soluble TNF-αR that has improved affinity, and thus is capable of acting as an antagonist for TNF activity. Nucleic acids that encode TNF-αR and exhibit sequence diversity, such as the natural diversity observed in cDNA libraries from activated T cells of human and other primates, are shuffled. The shuffled nucleic acids are expressed, *e.g.*, on phage, after which mutants are selected that bind to TNF-α with improved affinity. If desired, the improved mutants can be subjected to further assays using biological activity, and the shuffled genes can be subjected to one or more rounds of shuffling and screening.

Another target of interest for application of the methods of the invention is interferon-γ, and the evolution of antagonists of this cytokine. The receptor for IFN-γ consists of a binding component glycoprotein of 90 kD, a 228 amino acid extracellular portion, a transmembrane region, and a 222 amino acid intracellular region. Glycosylation is not required for functional activity. A single chain provides high affinity binding (10⁻⁹ -10⁻¹⁰ M), but is not sufficient for signaling. Receptor components dimerize upon ligand binding. The mouse IFN-γ receptor is 53% identical to that of mouse at the amino acid level. The human and mouse receptors only bind human and mouse IFN-γ, respectively. Vaccinia, cowpox and camelpox viruses have homologues of sIFN-γR, which have relatively low amino acid sequence similarity (~20%), but are capable of efficient neutralization of IFN-γ *in vitro.* These homologues bind human, bovine, rat (but not mouse) IFN-γ, and may have *in vivo* activity as IFN-γ antagonists. All eight cysteines are conserved in human, mouse, myxoma and Shope fibroma virus (6 in vaccinia virus) IFN-γR polypeptides, indicating similar 3-D structures. An extracellular portion of mIFN-γR with a kD of 100-300 pM has been expressed in insect cells. Treatment of NZB/W mice (a mouse model of human SLE) with msIFN-γ receptor (100 mg/three times a week i.p.) inhibits the onset of glomerulonephritis. All mice treated with sIFN-γ or anti-IFN-γ mAbs were alive 4 weeks after the treatment was discontinued, compared with 50% in a placebo group, and 78% of IFN-γ-treated mice died.

The methods of the invention can be used to evolve soluble IFN-γR receptor polypeptides with improved affinity, and to evolve IFN-γ with improved specific activity and improved capacity to activate cellular immune responses. In each case nucleic acids encoding the respective polypeptide, and which exhibit sequence diversity (*e.g.*, that observed in cDNA libraries from activated T cells from human and other primates), are subjected to recombination and screened to identify those recombinant nucleic acids that encode a polypeptide having improved activity. In the case of shuffled IFN-γR, the library of shuffled nucleic acids can be expressed on phage, which are screened to identify mutants that bind to IFN-γ with improved affinity. In the case of IFN-γ, the shuffled library is analyzed for improved specific activity and improved activation of the immune system, for example, by using activation of monocytes/macrophages as an assay. The evolved IFN-γ molecules can improve the efficacy of vaccinations (*e.g.* when used as adjuvants).

Diseases that can be treated using high-affinity sIFN-γR polypeptides obtained using the methods of the invention include, for example, multiple sclerosis, systemic lupus erythematosus (SLE), organ rejection after treatment, and graft versus host disease. Multiple sclerosis, for example, is characterized by increased expression of IFN-γ in the brain of the patients, and increased production of IFN-γ by patients' T cells *in vitro.* IFN-y treatment has been shown to significantly exacerbate the disease (in contrast to EAE in mice).

Transforming growth factor (TGF)-β is another cytokine that can be optimized for use in genetic vaccines using the methods of the invention. TGF-β has growth regulatory activities on essentially all cell types, and it has also been shown to have complex modulatory effects on the cells of the immune system. TGF-β inhibits proliferation of both B and T cells, and it also suppresses development of and differentiation of cytotoxic T cells and NK cells. TGF-β has been shown to direct IgA switching in both murine and human B cells. It was also shown to induce germline a transcription in murine and human B cells, supporting the conclusion that TGF-β can specifically induce IgA switching.

Due to its capacity to direct IgA switching, TGF-β is useful as a component of DNA vaccines which aim at inducing potent mucosal immunity, *e.g.* vaccines for diarrhea. Also, because of its potent anti-proliferative effects TGF-β is useful as a component of therapeutical cancer vaccines. TGF-β with improved specific activity and/or with improved expression levels/kinetics will have increased beneficial effects compared to the wild-type TGF-β.

Cytokines that can be optimized using the methods of the invention also include granulocyte colony stimulating factor (G-CSF) and granulocyte/macrophage colony stimulating factor (GM-CSF). These cytokines induce differentiation of bone marrow stem cell into granulocytes/macrophages. Administration of G-CSF and GM-CSF significantly improve recovery from bone marrow (BM) transplantation and radiotherapy, reducing infections and time the patients have to spend in hospitals. GM-CSF enhances antibody production following DNA vaccination. G-CSF is a 175 amino acid protein, while GM-CSF has 127 amino acids. Human G-CSF is 73% identical at the amino acid level to murine G-CSF and the two proteins show species cross-reactivity. G-CSF has a homodimeric receptor (dimeric with kD of~200 pM, monomeric ~ 2-4 nM), and the receptor for GM-CSF is a three subunit complex. Cell lines transfected with cDNA encoding G-CSF R proliferate in response to G-CSF. Cell lines dependent of GM-CSF available (such as TF-1). G-CSF is nontoxic and is presently working very well as a drug. However, the treatment is expensive, and more potent G-CSF might reduce the cost for patients and to the health care. Treatments with these cytokines are typically short-lasting and the patients are likely to never need the same treatment again reducing likelihood of problems with immunogenicity.

The methods of the invention are useful for evolving G-CSF and/or GM-CSF which have improved specific activity, as well as other polypeptides that have G-CSF and/or GM-CSF activity. G-CSF and/or GM-CSF nucleic acids having sequence diversity, *e.g.*, those obtained from cDNA libraries from diverse species, are shuffled to create a library of shuffled G-CSF and/or GM-CSF genes. These libraries can be screened by, for example, picking colonies, transfecting the plasmids into a suitable host cell (*e.g.,* CHO cells), and assaying the supernatants using receptor-positive cell lines. Alternatively, phage display or related techniques can be used, again using receptor-positive cell lines. Yet another screening method involves transfecting the shuffled genes into G-CSF/GM-CSF-dependent cell lines. The cells are grown one cell per well and/or at very low density in large flasks, and the cells that grow fastest are selected. Shuffled genes from these cells are isolated; if desired, these genes can be used for additional rounds of shuffling and selection.

Ciliary neurotrophic factor (CNTF) is another suitable target for application of the methods of the invention. CNTF has 200 amino acids which exhibit 80% sequence identity between rat and rabbit CNTF polypeptides. CNTF has IL-6-like inflammatory effects, and induces synthesis of acute phase proteins. CNTF is a cytosolic protein which belongs to the IL-6/IL-11/LIF/oncostatin M -family, and becomes biologically active only after becoming available either by cellular lesion or by an unknown release mechanism. CNTF is expressed by myelinating Schwann cells, astrocytes and sciatic nerves. Structurally, CNTF is a dimeric protein, with a novel anti-parallel arrangement of the subunits. Each subunit adopts a double crossover four-helix bundle fold, in which two helices contribute to the dimer interface. Lys-155 mutants lose activity, and some Glu-153 mutants have 5-10 higher biological activity. The receptor for CNTF consists of a specific CNTF receptor chain, gp130, and a LIF-β receptor. The CNTFR α-chain lacks a transmembrane domain portion, instead being GPI-anchored. At high concentration, CNTF can mediate CNTFR-independent responses. Soluble CNTFR binds CNTF and thereafter can bind to LIFR and induce signaling through gp130. CNTF enhances survival of several types of neurons, and protects neurons in an animal model of Huntington disease (in contrast to NGF, neurotrophic factor, and neurotrophin-3). CNTF receptor knockout mice have severe motor neuron deficits at birth, and CNTF knockout mice exhibit such deficits postnatally. CNTF also reduces obesity in mouse models. Decreased expression of CNTF is sometimes observed in psychiatric patients. Phase I studies in patients with ALS (annual incidence ~1/100 000, 5% familiar cases, 90% die within 6 years) found significant side effects after doses higher than 5 mg/kg/day subcutaneously (including anorexia, weight loss, reactivation of herpes simplex virus (HSV1), cough, increased oral secretions). Antibodies against CNTF were detected in almost all patients, thus illustrating the need for alternative CNTF with different immunological properties.

The recombination and screening methods of the invention can be used to obtain modified CNTF polypeptides that exhibit decreased immunogenicity *in vivo*; higher specific activity is also obtainable using the methods. Shuffling is conducted using nucleic acids encoding CNTF. In a preferred embodiment, an IL-6/LIF/(CNTF) hybrid is obtained by shuffling using an excess of oligonucleotides that encode to the receptor binding sites of CNTF. Phage display can then be used to test for lack of binding to the IL-6/LIF receptor. This initial screen is followed by a test for high affinity binding to the CNTF receptor, and, if desired, functional assays using CNTF responsive cell lines. The shuffled CNTF polypeptides can be tested to identify those that exhibit reduced immunogenicity upon administration to a mammal.

Another way in which the recombination and screening methods of the invention can be used to optimize CNTF is to improve secretion of the polypeptide. When a CNTF cDNA is operably linked to a leader sequence of hNGF, only 35-40 percent of the total CNTF produced is secreted.

Target diseases for treatment with optimized CNTF, using either the shuffled gene in an expression vector as in DNA vaccines, or a purified protein, include obesity, amyotrophic lateral sclerosis (ALS, Lou Gehrig's disease), diabetic neuropathy, stroke, and brain surgery.

Polynucleotides that encode chemokines can also be optimized using the methods of the invention and included in a genetic vaccine vector. At least three classes of chemokines are known, based on structure: C chemokines (such as lymphotactin), C-C chemokines (such as MCP-1, MCP-2, MCP-3, MCP-4, MIP-1a, MIP-1b, RANTES), C-X-C chemokines (such as IL-8, SDF-1, ELR, Mig, IP10) (Premack and Schall (1996) *Nature Med.* 2: 1174). Chemokines can attract other cells that mediate immune and inflammatory functions, thereby potentiating the immune response. Cells that are attracted by different types of chemokines include, for example, lymphocytes, monocytes and neutrophils. Generally, C-X-C chemokines are chemoattractants for neutrophils but not for monocytes, C-C chemokines attract monocytes and lymphocytes but not neutrophils, C chemokine attracts lymphocytes.

Genetic vaccine vectors can also include optimized recombinant polynucleotides that encode surface-bound accessory molecules, such as those that are involved in modulation and potentiation of immune responses. These molecules, which include, for example, B7-1 (CD80), B7-2 (CD86), CD40, ligand for CD40, CTLA-4, CD28, and CD150 (SLAM), can be subjected to DNA shuffling to obtain variants have altered and/or improved activities.

Optimized recombinant polynucleotides that encode CD1 molecules are also useful in a genetic vaccine vector for certain applications. CD1 are nonpolymorphic molecules that are structurally and functionally related to MHC molecules. Importantly, CD1 has MHC-like activities, and it can function as an antigen presenting molecule (Porcelli (1995) *Adv. Immunol.* 59: 1). CD1 is highly expressed on dendritic cells, which are very efficient antigen presenting cells. Simultaneous transfection of target cells with DNA vaccine vectors encoding CD1 and an antigen of interest is likely to boost the immune response. Because CD1 cells, in contrast to MHC molecules, exhibit limited allelic diversity in an outbred population (Porcelli, *supra*.)*,* large populations of individuals with different genetic backgrounds can be vaccinated with one CD1 allele. The functional properties of CD1 molecules can be improved by the DNA shuffling methods of the invention.

Optimized recombinant TAP genes and/or gene products can also be included in a genetic vaccine vector. TAP genes and their optimization for various purposes are discussed in more detail below. Moreover, heat shock proteins (HSP), such as HSP70, can also be evolved for improved presentation and processing of antigens. HSP70 has been shown to act as adjuvant for induction of CD8⁺ T cell activation and it enhances immunogenicity of specific antigenic peptides (Blachere *et al.* (1997) *J. Exp. Med.* 186:1315-22). When HSP70 is encoded by a genetic vaccine vector, it is likely to enhance presentation and processing of antigenic peptides and thereby improve the efficacy of the genetic vaccines. DNA shuffling can be used to further improve the properties, including adjuvant activity, of heat shock proteins, such as HSP70.

Recombinantly produced cytokine, chemokine, and accessory molecule polypeptides, as well as antagonists of these molecules, can be used to influence the type of immune response to a given stimulus. However, the administration of polypeptides sometimes has shortcomings, including short half life, high expense, difficult to store (must be stored at 4°C), and a requirement for large volumes. Also, bolus injections can sometimes cause side effects. Administration of polynucleotides that encode the recombinant cytokines or other molecules overcomes most or all of these problems. DNA, for example, can be prepared in high purity, is stable, temperature resistant, noninfectious, easy to manufacture. In addition, polynucleotide-mediated administration of cytokines can provide long-lasting, consistent expression, and administration of polynucleotides in general is regarded as being safe.

The functions of cytokines, chemokines and accessory molecules are redundant and pleiotropic, and therefore can be difficult to determine which cytokines or cytokine combinations are the most potent in inducing and enhancing antigen specific immune responses following vaccination. Furthermore, the most useful combination of cytokines and accessory molecules is typically different depending on the type of immune response that is desired following vaccination. As an example, IL-4 has been shown to direct differentiation of T_{H}2 cells (which produce high levels of IL-4, IL-5 and IL-13, and mediate allergic immune responses), whereas IFN-γ and IL-12 direct differentiation of T_{H}1 cells (which produce high levels of IL-2 and IFN-γ), and mediate delayed type immune responses. Moreover, the most useful combination of cytokines and accessory molecules is also likely to depend on the antigen used in the vaccination. The invention provides a solution to this problem of obtaining an optimized genetic vaccine cocktail. Different combinations of cytokines, chemokines and accessory molecules are assembled into vectors using the methods described herein. These vectors are then screened for their capacity to induce immune responses *in vivo* and *in vitro.*

Large libraries of vectors, generated by gene shuffling and combinatorial molecular biology, are screened for maximal capacity to direct immune responses towards, for example, a T_{H}1 or T_{H}2 phenotype, as desired. A library of different vectors can be generated by assembling different evolved promoters, (evolved) cytokines, (evolved) cytokine antagonists, (evolved) chemokines, (evolved) accessory molecules and immunostimulatory sequences, each of which can be prepared using methods described herein. DNA sequences and compounds that facilitate the transfection and expression can be included. If the pathogen(s) is known, specific DNA sequences encoding immunogenic antigens from the pathogen can be incorporated into these vectors providing protective immunity against the pathogen(s) (as in genetic vaccines).

Initial screening is preferably carried out *in vitro.* For example, the library can be introduced into cells which are tested for ability to induce differentiation of T cells capable of producing cytokines that are indicative of the type of immune response desired. For a T_{H}1 response, for example, the library is screened to identify recombinant polynucleotides that are capable of inducing T cells to produce IL-2 and IFN-γ, while screening for induction of T cell production of IL-4, IL-5, and IL-13 is performed to identify recombinant polynucleotides that favor a T_{H}2 response.

Screening can also be conducted *in vivo,* using animal models. For example, vectors produced using the methods of the invention can be tested for ability to protect against a lethal infection. Another screening method involves injection of *Leishmania major* parasites into footpads of BALB/c mice (nonhealer). Pools of plasmids are injected i.v., i.p. or into footpads of these mice and the size of the footpad swelling is followed. Yet another *in vivo* screening method involves detection of IgE levels after infection with *Nippostrongylus brasiliensis.* High levels indicate a T_{H}2 response, while low levels of IgE indicate a T_{H}1 response.

Successful results in animal models are easy to verify in humans. *In vitro* screening can be conducted to test for human T_{H}1 or T_{H}2 phenotype, or for other desired immune response. Vectors can also be tested for ability to induce protection against infection in humans.

Because the principles of immune functions are similar in a wide variety of infections, immunostimulating DNA vaccine vectors may not only be useful in the treatment of a number of infectious diseases but also in prevention of the infections, when the vectors are delivered to the sites of the entry of the pathogen (*e.g.*, the lung or gut).

### 3. Agonists or antagonists of cellular receptors

The invention also provides methods for obtaining optimized recombinant polynucleotides that encode a peptide or polypeptide that can interact with a cellular receptor that is involved in mediating an immune response. The optimized recombinant polynucleotides can act as an agonist or an antagonist of the receptor.

Cytokine antagonists can be used as components of genetic vaccine cocktails. Blocking immunosuppressive cytokines, rather than adding single proinflammatory cytokines, is likely to potentiate the immune response in a more general manner, because several pathways are potentiated at the same time. By appropriate choice of antagonist, one can tailor the immune response induced by a genetic vaccine in order to obtain the response that is most effective in achieving the desired effect. Antagonists against any cytokine can be used as appropriate; particular cytokines of interest for blocking include, for example, IL-4, IL-13, IL-10, and the like.

The invention provides methods of obtaining cytokine antagonists that exhibit greater effectiveness in blocking the action of the respective cytokine. Polynucleotides that encode improved cytokine antagonists can be obtained by using gene shuffling to generate a recombinant library of polynucleotides which are then screened to identify those that encode an improved antagonist. As substrates for the DNA shuffling, one can use, for example, polynucleotides that encode receptors for the respective cytokine. At least two forms of the substrate will be present in the recombination reaction, with each form differing from the other in at least one nucleotide position. In a preferred embodiment, the different forms of the polynucleotide are homologous cytokine receptor genes from different organisms. The resulting library of recombinant polynucleotides is then screened to identify those that encode cytokine antagonists with the desired affinity and biological activity.

As one example of the type of effect that one can achieve by including a cytokine antagonist in a genetic vaccine cocktail, as well as how the effect can be improved using the DNA shuffling methods of the invention, IL-10 is discussed. The same rationale can be applied to obtaining and using antagonists of other cytokines. Interleukin-10 (IL-10) is perhaps the most potent anti-inflammatory cytokine known to date. IL-10 inhibits a number of pathways that potentiate inflammatory responses. The biological activities of IL-10 include inhibition of MHC class II expression on monocytes, inhibition of production of IL-1, IL-6, IL-12, TNF-α by monocytes/macrophages, and inhibition of proliferation and IL-2 production by T lymphocytes. The significance of IL-10 as a regulatory molecule of immune and inflammatory responses was clearly demonstrated in IL-10 deficient mice. These mice are growth-retarded, anemic and spontaneously develop an inflammatory bowel disease (Kuhn *et al.* (1993) *Cell* 75: 263). In addition, both innate and acquired immunity to *Listeria monocytogenes* were shown to be elevated in IL-10 deficient mice (Dai *et al.* (1997) *J. Immunol.* 158: 2259). It has also been suggested that genetic differences in the levels of IL-10 production may affect the risk of patients to die from complications meningococcal infection. Families with high IL-10 production had 20-fold increased risk of fatal outcome of meningococcal disease (Westendorp *et al.* (1997) *Lancet* 349: 170).

IL-10 has been shown to activate normal and malignant B cells *in vitro,* but it does not appear to be a major growth promoting cytokine for normal B cells *in vivo,* because IL-10 deficient mice have normal levels of B lymphocytes and Ig in their circulation. In fact, there is evidence that IL-10 can indirectly downregulate B cell function through inhibition of the accessory cell function of monocytes. However, IL-10 appears to play a role in the growth and expansion of malignant B cells. Anti-IL-10 monoclonal antibodies and IL-10 antisense oligonucleotides have been shown to inhibit transformation of B cells by EBV *in vitro.* In addition, B cell lymphomas are associated with EBV and most EBV⁺ lymphomas produce high levels of IL-10, which is derived both from the human gene and the homologue of IL-10 encoded by EBV. AIDS-related B cell lymphomas also secrete high levels of IL-10. Furthermore, patients with detectable serum IL-10 at the time of diagnosis of intermediate/high-grade non-Hodgkin's lymphoma have short survival, further suggesting a role for IL-10 in the pathogenesis of B cell malignancies.

Antagonizing IL-10 *in vivo* can be beneficial in several infectious and malignant diseases, and in vaccination. The effect of blocking of IL-10 is an enhancement of immune responses that is independent of the specificity of the response. This is useful in vaccinations and in the treatment of serious infectious diseases. Moreover, an IL-10 antagonist is useful in the treatment of B cell malignancies which exhibit overproduction of IL-10 and viral IL-10, and it may also be useful in boosting general anti-tumor immune response in cancer patients. Combining an IL-10 antagonist with gene therapy vectors may be useful in gene therapy of tumor cells in order to obtain maximal immune response against the tumor cells. If shuffling of IL-10 results in IL-10 with improved specific activity, this IL-10 molecule would have potential in the treatment of autoimmune diseases and inflammatory bowel diseases. IL-10 with improved specific activity may also be useful as a component of gene therapy vectors in reducing the immune response against vectors which are recognized by memory cells and it may also reduce the immunogenicity of these vectors.

An antagonist of IL-10 has been made by generating a soluble form of IL-10 receptor (sIL-10R; Tan *et al.* (1995) *J. Biol. Chem.* 270: 12906). However, sIL-10R binds IL-10 with Kd of 560 pM, whereas the wild-type, surface-bound receptor has affinity of 35-200 pM. Consequently, 150-fold molar excess of sIL-10R is required for half-maximal inhibition of biological function of IL-10. Moreover, affinity of viral IL-10 (IL-10 homologue encoded by Epstein-Barr virus) to sIL-10R is more than 1000 fold less than that of hIL-10, and in some situations, such as when treating EBV-associated B cell malignancies, it may be beneficial if one can also block the function of viral IL-10. Taken together, this soluble form of IL-10R is unlikely to be effective in antagonizing IL-10 *in vivo.*

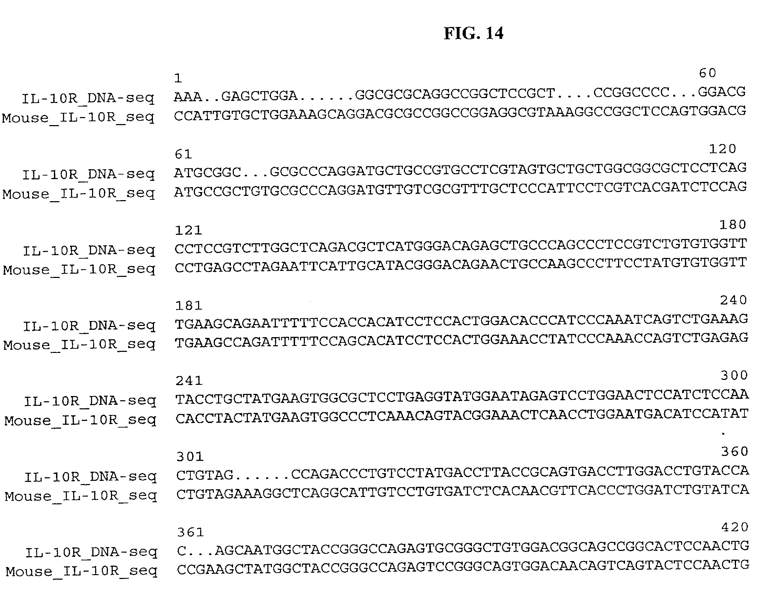

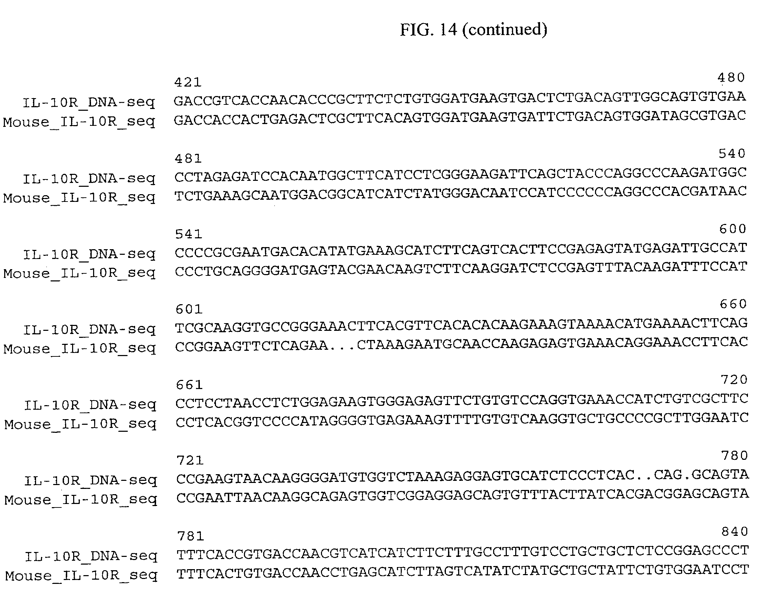

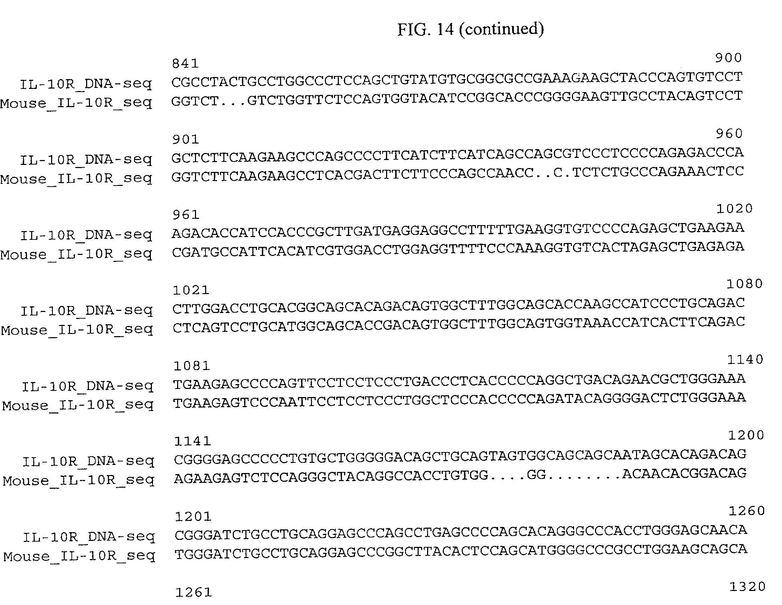

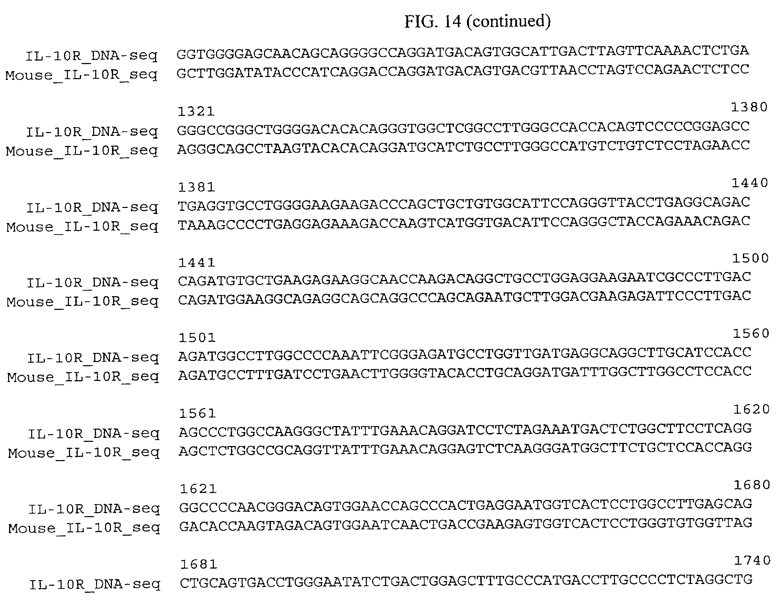

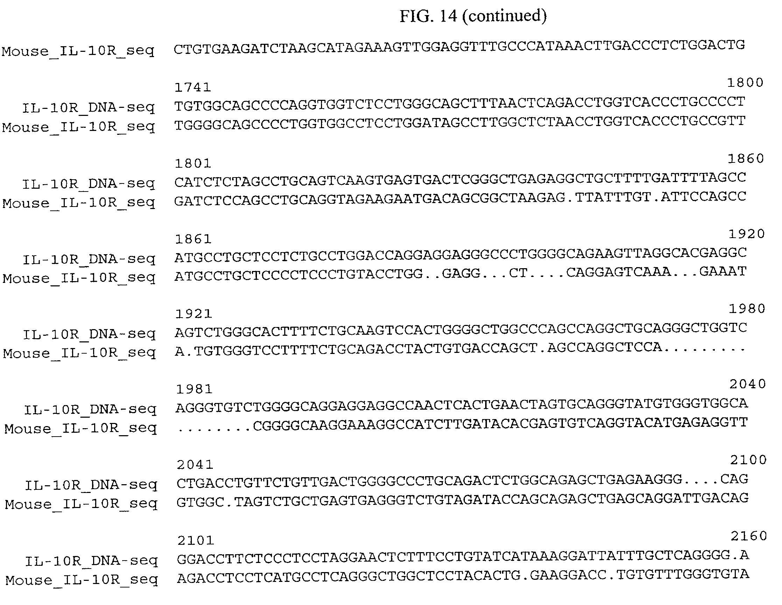

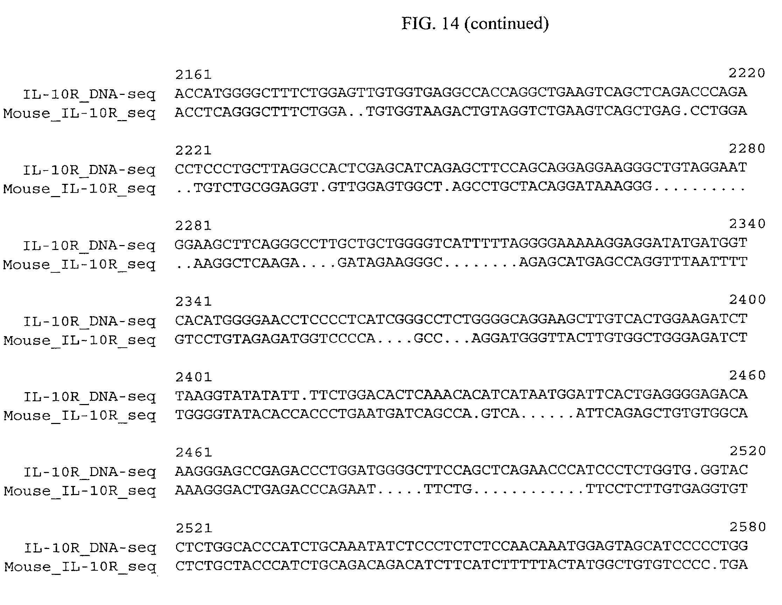

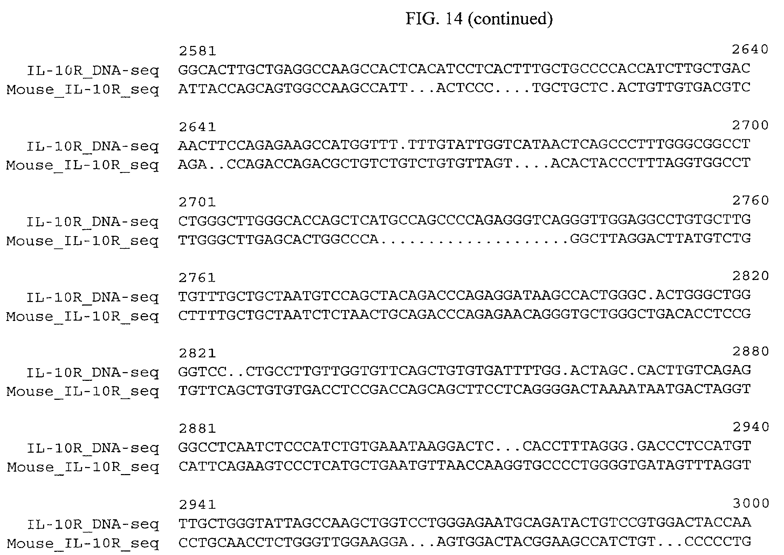

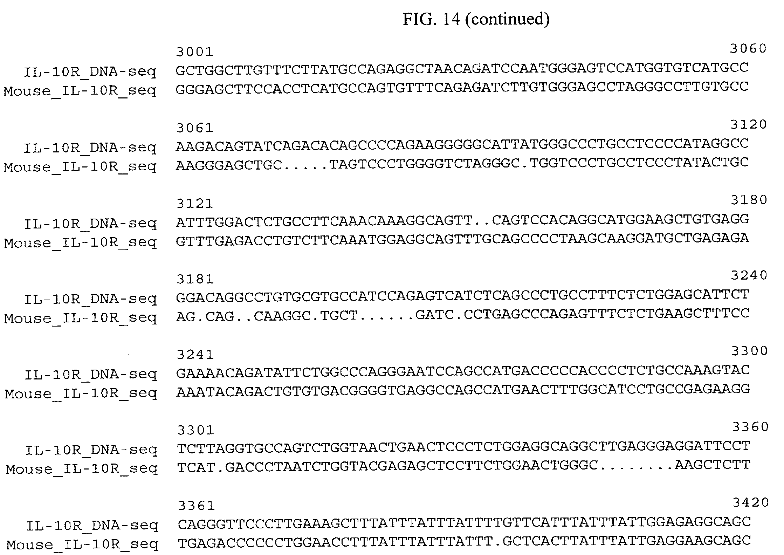

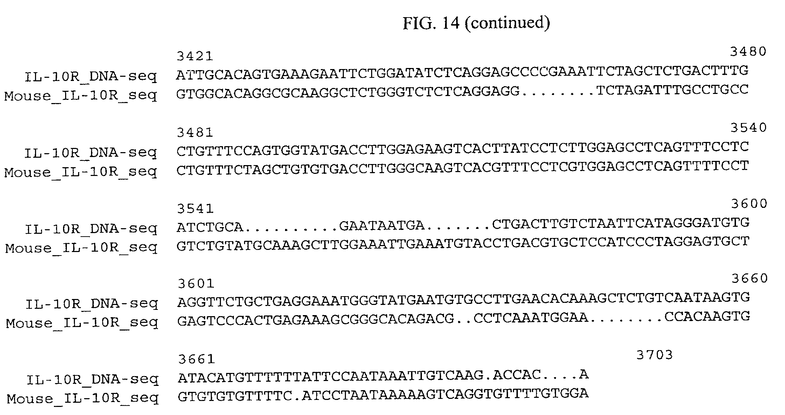

To obtain an IL-10 antagonist that has sufficient affinity and antagonistic activity to function *in vivo*, DNA shuffling can be performed using polynucleotides that encode IL-10 receptor. IL-10 receptor with higher than normal affinity will function as an IL-10 antagonist, because it strongly reduces the amount of IL-10 available for binding to functional, wild-type IL-1 OR. In a preferred embodiment, IL-10R is shuffled using homologous cDNAs encoding IL-10R derived from human and other mammalian species. An alignment of human and mouse IL-10 receptor sequences is shown in Figure 14 to illustrate the feasibility of family DNA shuffling when evolving IL-10 receptors with improved affinity. A phage library of IL-10 receptor recombinants can be screened for improved binding of shuffled IL-10R to human or viral IL-10. Wild-type IL-10 and/or viral IL-10 are added at increasing concentrations to demand for higher affinity. Phage bound to IL-10 can be recovered using anti-IL-10 monoclonal antibodies. If desired, the shuffling can be repeated one or more times, after which the evolved soluble IL-10R is analyzed in functional assays for its capacity to neutralize the biological activities of IL-10/viral IL-10. More specifically, evolved soluble IL-10R is studied for its capacity to block the inhibitory effects of IL-10 on cytokine synthesis and MHC class II expression by monocytes, proliferation by T cells, and for its capacity to inhibit the enhancing effects of IL-10 on proliferation ofB cells activated by anti-CD40 monoclonal antibodies.

An IL-10 antagonist can also be generated by evolving IL-10 to obtain variants that bind to IL-10R with higher than wild-type affinity, but without receptor activation. The advantage of this approach is that one can evolve an IL-10 molecule with improved specific activity using the same methods. In a preferred embodiment, IL-10 is shuffled using homologous cDNAs encoding IL-10 derived from human and other mammalian species. In addition, a gene encoding viral IL-10 can be included in the shuffling. A library of IL-10 recombinants is screened for improved binding to human IL-10 receptor. Library members bound to IL-10R can be recovered by anti-IL-10R monoclonal antibodies. This screening protocol is likely to result in IL-10 molecules with both antagonistic and agonistic activities. Because initial screen demands for higher affinity, a proportion of the agonists are likely to have improved specific activity when compared to wild-type human IL-10. The functional properties of the mutant IL-10 molecules are determined in biological assays similar to those described above for ultrahigh-affinity IL-10 receptors (cytokine synthesis and MHC class II expression by monocytes, proliferation of B and T cells). An antagonistic IL-4 mutant has been previously generated illustrating the general feasibility of the approach (Kruse *et al.* (1992) *EMBO J.* 11: 3237-3244). One amino acid mutation in IL-4 resulted in a molecule that efficiently binds to IL-4R α-chain but has minimal IL-4-like agonistic activity.

Another example of an IL-10 antagonist is IL-20/mda-7, which is a 206 amino acid secreted protein. This protein was originally characterized as mda-7, which is a melanoma cell-derived negative regulator of tumor cell growth (Jiang *et al.* (1995) *Oncogene* 11: 2477; (1996) *Proc. Nat'l. Acad. Sci. USA* 93: 9160). IL-20/mda-7 is structurally related to IL-10, and it antagonizes several functions of IL-10 (Abstract of the 13th European Immunology Meeting, Amsterdam, 22-25 June 1997). In contrast to IL-10, IL-20/mda-7 enhances expression of CD80 (B7-1) and CD86 (B7-2) on human monocytes and it upregulates production of TNF-α and IL-6. IL-20/mda-7 also enhances production of IFN-γ by PHA-activated PBMC. The invention provides methods of improving genetic vaccines by incorporation of IL-20/mda-7 genes into the genetic vaccine vectors. The methods of the invention can be used to obtain IL-20/mda-7 variants that exhibit improved ability to antagonize IL-10 activity.

When a cytokine antagonist is used as a component of DNA vaccine or gene therapy vectors, maximal local effect is desirable. Therefore, in addition to a soluble form of a cytokine antagonist, a transmembrane form of the antagonist can be generated. The soluble form can be given in purified polypeptide form to patients by, for example, intravenous injection. Alternatively, a polynucleotide encoding the cytokine antagonist can be used as a component as a component of a genetic vaccine or a gene therapy vector. In this case, either or both of the soluble and transmembrane forms can be used. Where both soluble and transmembrane forms of the antagonist are encoded by the same vector, the target cells express both forms, resulting in maximal inhibition of cytokine function on the target cell surface and in their immediate vicinity.

The peptides or polypeptides obtained using these methods can substitute for the natural ligands of the receptors, such as cytokines or other costimulatory molecules in their ability to exert an effect on the immune system via the receptor. A potential disadvantage of administering cytokines or other costimulatory molecules themselves is that an autoimmune reaction could be induced against the natural molecule, either due to breaking tolerance (if using a natural cytokine or other molecule) or by inducing cross-reactive immunity (humoral or cellular) when using related but distinct molecules. Through using the methods of the invention, one can obtain agonists or antagonists that avoid these potential drawbacks. For example, one can use relatively small peptides as agonists that can mimic the activity of the natural immunomodulator, or antagonize the activity, without inducing cross-reactive immunity to the natural molecule. In a presently preferred embodiment, the optimized agonist or antagonist obtained using the methods of the invention is about 50 amino acids or length or less, more preferably about 30 amino acids or less, and most preferably is about 20 amino acids in length, or less. The agonist or antagonist peptide is preferably at least about 4 amino acids in length, and more preferably at least about 8 amino acids in length. Polynucleotides that flank the coding sequence of the mimetic peptide can also be optimized using the methods of the invention in order to optimize the expression, conformation, or activity of the mimetic peptide.

The optimized agonist or antagonist peptides or polypeptides are obtained by generating a library of recombinant polynucleotides and screening the library to identify those that encode a peptide or polypeptide that exhibits an enhanced ability to modulate an immune response. The library can be produced using methods such as DNA shuffling or other methods described herein or otherwise known to those of skill in the art. Screening is conveniently conducted by expressing the peptides encoded by the library members on the surface of a population of replicable genetic packages and identifying those members that bind to a target of interest, *e.g.,* a receptor.

The optimized recombinant polynucleotides that are obtained using the methods of the invention can be used in several ways. For example, the polynucleotide can be placed in a genetic vaccine vector, under the control of appropriate expression control sequences, so that the mimetic peptide is expressed upon introduction of the vector into a mammal. If desired, the polynucleotide can be placed in the vector embedded in the coding sequence of the surface protein (*e.g.*, geneIII or geneVIII) in order to preserve the conformation of the mimetic. Alternatively, the mimetic-encoding polynucleotide can be inserted directly into the antigen-encoding sequence of the genetic vaccine to form a coding sequence for a "mimotope-on-antigen" structure. The polynucleotide that encodes the mimotope-on-antigen structure can be used within a genetic vaccine, or can be used to express a protein that is itself administered as a vaccine. As one example of this type of application, a coding sequence of a mimetic peptide is introduced into a polynucleotide that encodes the "M-loop" of the hepatitis B surface antigen (HBsAg) protein. The M-loop is a six amino acid peptide sequence bounded by cysteine residues, which is found at amino acids 139-147 (numbering within the S protein sequence). The M-loop in the natural HBsAg protein is recognized by the monoclonal antibody RFHB7 (Chen *et al., Proc. Nat'l. Acad. Sci. USA,* 93: 1997-2001 (1996)). According to Chen *et al.,* the M-loop forms an epitope of the HBsAg that is non-overlapping and separate from at least four other HBsAg epitopes.

Because of the probable Cys-Cys disulfide bond in this hydrophilic part of the protein, amino acids 139-147 are likely in a cyclic conformation. This structure is therefore similar to that found in the regions of the filamentous phage proteins pIII and pVIII where mimotope sequences are placed. Therefore, one can insert a mimotope obtained using the methods of the invention into this region of the HBsAg amino acid sequence.

The chemokine receptor CCR6 is an example of a suitable target for a peptide mimetic obtained using the methods. The CCR6 receptor is a 7-transmembrane domain protein (Dieu *et al., Biochem. Biophys. Res. Comm.* 236: 212-217 (1997) and *J. Biol. Chem.* 272: 14893-14898 (1997)) that is involved in the chemoattraction of immature dendritic cells, which are found in the blood and migrate to sites of antigen uptake (Dieu *et al., J. Exp. Med.* 188: 373-386 (1998)). CCR6 binds the chemokine MIP-3α, so a mimetic peptide that is capable of activating CCR6 can provide a further chemoattractant function to a given antigen and thus promote uptake by dendritic cells after immunization with the antigen antigen-mimetic fusion or a DNA vector that expresses the antigen.

Another application of this method of the invention is to obtain molecules that can act as an agonist for the macrophage scavenger receptor (MSR; *see,* Wloch *et al., Hum. Gene Ther.* 9: 1439-1447 (1998)). The MSR is involved in mediating the effects of various immunomodulators. Among these are bacterial DNA, including the plasmids used in DNA vaccination, and oligonucleotides, which are often potent immunostimulators. Oligonucleotides of certain chemical structure (*e.g.*, phosphothio-oligonucleotides) are particularly potent, while bacterial or plasmid DNA must be used in relatively large quantities to produce an effect. Also mediated by the MSR is the ability of oligonucleotides that contain dG residues to stimulate B cells and enhance the activity of immunostimulatory CpG motifs, and of lipopolysaccharides to activate macrophages. Some of these activities are toxic. Each of these immunomodulators, along with a variety of polyanionic ligands, binds to the MSR. The methods of the invention can be used to obtain mimetics of one or more of these immunomodulators that bind to the MSR with high affinity but are devoid of toxic properties. Such mimetic peptides are useful as immunostimulators or adjuvants.

The MSR is a trimeric integral membrane glycoprotein. The three extracellular C-terminal cysteine-rich regions are connected to the transmembrane domain by a fibrous region that is composed of an α-helical coil and a collagen-like triple helix (*see,* Kodama *et al., Nature* 343: 531-535 (1990)). Therefore, screening of the library of recombinant polynucleotides can be accomplished by expressing the extracellular receptor structure and artificially attaching it to plastic surfaces. The libraries can be expressed, *e.g.*, by phage display, and screened to identify those that bind to the receptors with high affinity. The optimized recombinant polynucleotides identified by this method can be incorporated into antigen-encoding sequences to evaluate their modulatory effect on the immune response.

### 4. Costimulatory molecules capable of inhibiting or enhancing activation, differentiation, or anergy of antigen-specific T cells

Also provided are methods of obtaining optimized recombinant polynucleotides that, when expressed, are capable of inhibiting or enhancing the activation, differentiation, or anergy of antigen-specific T cells. T cell activation is initiated when T cells recognize their specific antigenic peptides in the context of MHC molecules on the plasma membrane of antigen presenting cells (APC), such as monocytes, dendritic cells (DC), Langerhans cells or B cells. Activation of CD4⁺ T cells requires recognition by the T cell receptor (TCR) of an antigenic peptide in the context of MHC class II molecules, whereas CD8⁺ T cells recognize peptides in the context of MHC class I molecules. Importantly, however, recognition of the antigenic peptides is not sufficient for induction of T cell proliferation and cytokine synthesis. An additional costimulatory signal, "the second signal", is required. The costimulatory signal is mediated via CD28, which binds to its ligands B7-1 (CD80) or B7-2 (CD86), typically expressed on the antigen presenting cells. In the absence of the costimulatory signal, no T cell activation occurs, or T cells are rendered anergic. In addition to CD28, CTLA-4 (CD 152) also functions as a ligand for B7-1 and B7-2. However, in contrast to CD28, CTLA-4 mediates a negative regulatory signal to T cells and/or to induce anergy and tolerance (Walunas *et al.* (1994) *Immunity* 1: 405; Karandikar *et al.* (1996) *J. Exp. Med.* 184: 783).

B7-1 and B7-2 have been shown to be able to regulate several immunological responses, and they have been implicated to be of importance in the immune regulation in vaccinations, allergy, autoimmunity and cancer. Gene therapy and genetic vaccine vectors expressing B7-1 and/or B7-2 have also been shown to have therapeutic potential in the treatment of the above mentioned diseases and in improving the efficacy of genetic vaccines.

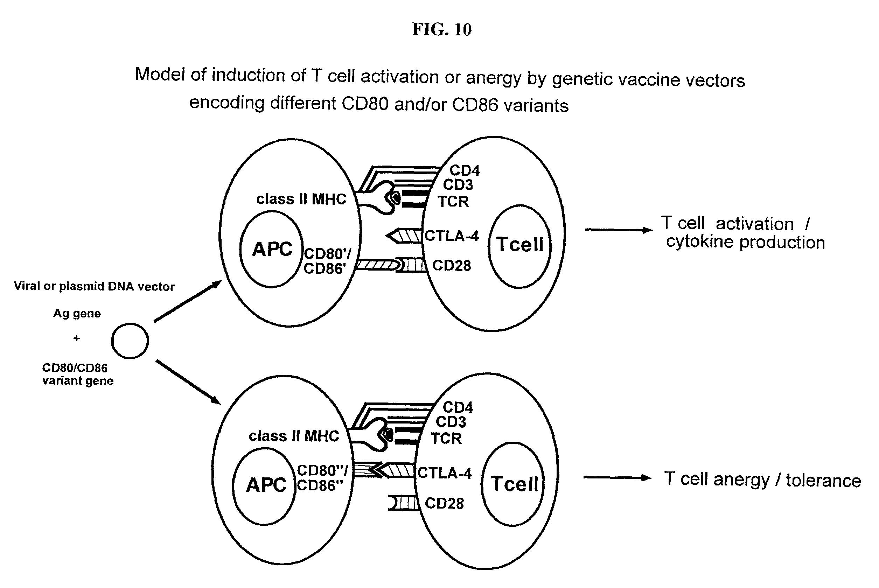

Figure 10 illustrates interaction of APC and CD4⁺ T cells, but the same principle is true with CD8⁺ T cells, with the exception that the T cells recognize the antigenic peptides in the context of MHC class I molecules. Both B7-1 and B7-2 bind to CD28 and CTLA-4, even though the sequence similarities between these four molecules are very limited (20-30%). It is desirable to obtain mutations in B7-1 and B7-2 that only influence binding to one ligand but not to the other, or improve activity through one ligand while decreasing the activity through the other. Moreover, because the affinities of B7 molecules to their ligands appear to be relatively low, it would also be desirable to find mutations that improve/alter the activities of the molecules. However, rational design does not enable predictions of useful mutations because of the complexity of the molecules.

The invention provides methods of overcoming these difficulties, enabling one to generate and identify functionally different B7 molecules with altered relative capacities to induce T cell activation, differentiation, cytokine production, anergy and/or tolerance. Through use of the methods of the invention, one can find mutations in B7-1 and B7-2 that only influence binding to one ligand but not to the other, or that improve activity through one ligand while decreasing the activity through the other. DNA shuffling is likely to be the most powerful method in discovering new B7 variants with altered relative binding capacities to CD28 and CTLA-4. B7 variants which act through CD28 with improved activity (and with decreased activity through CTLA-4) are expected to have improved capacity to induce activation of T cells. In contrast, B7 variants which bind and act through CTLA-4 with improved activity (and with decreased activity through CD28) are expected to be potent negative regulators of T cell functions and to induce tolerance and anergy.

DNA shuffling or other recombination method is used to generate B7 (*e.g.*, B7-1/CD80 and B7-2/CD86) variants which have altered relative capacity to act through CD28 and CTLA-4 when compared to wild-type B7 molecules. In a preferred embodiment, the different forms of substrate used in the recombination reaction are B7 cDNAs from various species. Such cDNAs can be obtained by methods known to those of skill in the art, including RT-PCR.

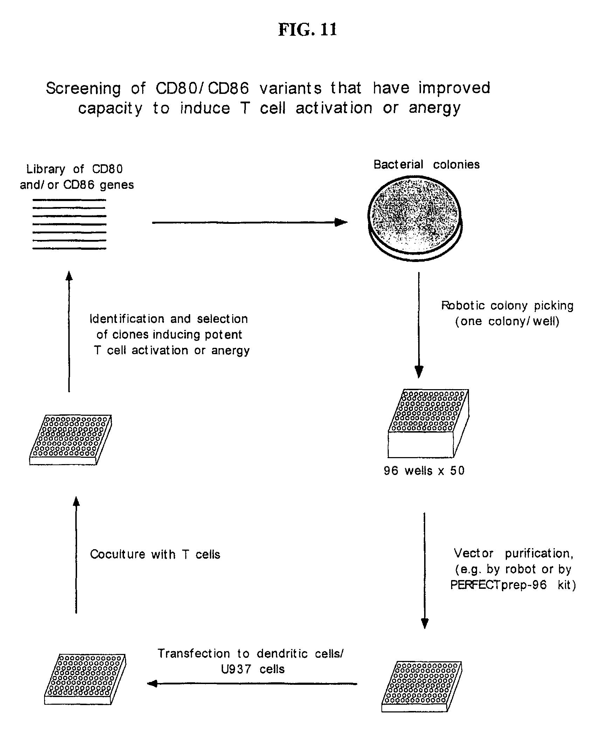

Typically, genes encoding these variant B7 molecules are incorporated into genetic vaccine vectors encoding an antigen, so that one the vectors can be used to modify antigen-specific T cell responses. Vectors that harbor B7 genes that efficiently act through CD28 are useful in inducing, for example, protective immune responses, whereas vectors that harbor genes encoding B7 genes that efficiently act through CTLA-4 are useful in inducing, for example, tolerance and anergy of allergen- or autoantigen-specific T cells. In some situations, such as in tumor cells or cells inducing autoimmune reactions, the antigen may already be present on the surface of the target cell, and the variant B7 molecules may be transfected in the absence of additional exogenous antigen gene. Figure 11 illustrates a screening protocol that one can use to identify B7-1 (CD80) and/or B7-2 (CD86) variants that have increased capacity to induce T cell activation or anergy, and the application of this strategy is described in more detail in Example 1.

Several approaches for screening of the variants can be taken. For example, one can use a flow cytometry-based selection systems. The library of B7-1 and B7-2 molecules is transfected into cells that normally do not express these molecules (*e.g.*, COS-7 cells or any cell line from a different species with limited or no cross-reactivity with man regarding B7 ligand binding). An internal marker gene can be incorporated in order to analyze the copy number per cell. Soluble CTLA-4 and CD28 molecules can be generated to for use in the flow cytometry experiments. Typically, these will be fused with the Fc portion of IgG molecule to improve the stability of the molecules and to enable easy staining by labeled anti-IgG mAbs, as described by van der Merwe *et al.* (J. Exp. Med. 185: 393, 1997). The cells transfected with the library of B7 molecules are then stained with the soluble CTLA-4 and CD28 molecules. Cells demonstrating increased or decreased CTLA-4/CD28 binding ratio will be sorted. The plasmids are then recovered and the shuffled B7 variant-encoding sequences identified. These selected B7 variants can then be subjected to new rounds of shuffling and selection, and/or they can be further analyzed using functional assays as described below.

The B7 variants can also be directly selected based on their functional properties. For *in vivo* studies, the B7 molecules can also be evolved to function on mouse cells. Bacterial colonies with plasmids with mutant B7 molecules are picked and the plasmids are isolated. These plasmids are then transfected into antigen presenting cells, such as dendritic cells, and the capacities of these mutants to activate T cells is analyzed. One of the advantages of this approach is that no assumptions on the binding affinities or specificities to the known ligands are made, and possibly new activities through yet to be identified ligands can be found. In addition to dendritic cells, other cells that are relatively easy to transfect (*e.g.*, U937 or COS-7) can be used in the screening, provided that the "first T cell signal" is induced by, for example, anti-CD3 monoclonal antibodies. T cell activation can be analyzed by methods known to those of skill in the art, including, for example, measuring proliferation, cytokine production, CTL activity or expression of activation antigens such as IL-2 receptor, CD69 or HLA-DR molecules. Usage of antigen-specific T cell clones, such as T cells specific for house dust mite antigen Der p I, will allow analysis of antigen-specific T cell activation (Yssel *et al.* (1992) *J*. *Immunol.* 148: 738-745). Mutants are identified that can enhance or inhibit T cell proliferation or enhance or inhibit CTL responses. Similarly variants that have altered capacity to induce cytokine production or expression of activation antigens as measured by, for example, cytokine-specific ELISAs or flow cytometry can be identified.

The B7 variants are useful in modulating immune responses in autoimmune diseases, allergy, cancer, infectious disease and vaccination. B7 variants which act through CD28 with improved activity (and with decreased activity through CTLA-4) will have improved capacity to induce activation of T cells. In contrast, B7 variants which bind and act through CTLA-4 with improved activity (and with decreased activity through CD28) will be potent negative regulators of T cell functions and to induce tolerance and anergy. Thus, by incorporating genes encoding these variant B7 molecules into genetic vaccine vectors encoding an antigen, it is possible to modify antigen-specific T cell responses. Vectors that harbor B7 genes that efficiently act through CD28 are useful in inducing, for example, protective immune responses, whereas vectors that harbor genes encoding B7 genes that efficiently act through CTLA-4 are useful in inducing, for example, tolerance and anergy of allergen- or autoantigen-specific T cells. In some situations, such as in tumor cells or cells inducing autoimmune reactions, the antigen may already be present on the surface of the target cell, and the variant B7 molecules may be transfected in the absence of additional exogenous antigen gene.

The methods of the invention are also useful for obtaining B7 variants that have increased effectiveness in directing either T_{H}1 or T_{H}2 cell differentiation. Differential roles have been observed for B7-1 and B7-2 molecules in the regulation ofT helper (T_{H}) cell differentiation (Freeman *et al.* (1995) *Immunity* 2: 523; Kuchroo *et al.* (1995) *Cell* 80: 707). T_{H} cell differentiation can be measured by analyzing the cytokine production profiles induced by each particular variant. High levels of IL-4, IL-5 and /or IL-13 are an indication of efficient T_{H}2 cell differentiation whereas high levels of IFN-γ or IL-2 production can be used as a marker of T_{H}1 cell differentiation. B7 variants with altered capacity to induce T_{H}1 or T_{H}2 cell differentiation are useful, for example, in the treatment of allergic, malignant, autoimmune and infectious diseases and in vaccination.

Also provided by the invention are methods of obtaining B7 variants that have enhanced capacity to induce IL-10 production by antigen-specific T cells. Elevated production of IL-10 is a characteristic of regulatory T cells, which can suppress proliferation of antigen-specific CD4⁺ T cells (Groux *et al.* (1997) *Nature* 389: 737). DNA shuffling is performed as described above, after which recombinant nucleic acids encoding B7 variants having enhanced capability of inducing IL-10 0 can be identified by, for example, ELISA or flow cytometry using intracytoplasmic cytokine staining. The variants that induce high levels of IL-10 production are useful in the treatment of allergic and autoimmune diseases.

### D. Optimization of Transport and Presentation of Antigens

The invention also provides methods of obtaining genetic vaccines and accessory molecules that can improve the transport and presentation of antigenic peptides. A library of recombinant polynucleotides is created and screened to identify those that encode molecules that have improved properties compared to the wild-type counterparts. The polynucleotides themselves can be used in genetic vaccines, or the gene products of the polynucleotides can be utilized for therapeutic or prophylactic applications.

### 1. Proteasomes

The class I peptides presented on major histocompatibility complex molecules are generated by cellular proteasomes. Interferon-gamma can stimulate antigen presentation, and part of the mechanism of action of interferon may be due to induction of the proteasome beta-subunits LMP2 and LMP7, which replace the homologous beta-subunits Y (delta) and X (epsilon). Such a replacement changes the peptide cleavage specificity of the proteasome and can enhance class I epitope immunogenicity. The Y (delta) and X (epsilon) subunits, as well as other recently discovered proteasome subunits such as the MECL-1 homologue MC14, are characteristic of cells which are not specialized in antigen presentation. Thus, the incorporation into cells by DNA transfer of LMP2, LMP7, MECL-1 and/or other epitope presentation-specific and potentially interferon-inducible subunits can enhance epitope presentation. It is likely that the peptides generated by the proteasome containing the interferon-inducible subunits are transported to the endoplasmic reticulum by the TAP molecules.

The invention provides methods of obtaining proteasomes that exhibit increased or decreased ability to specifically process MHC class I epitopes. According to the methods, DNA shuffling is used to obtain evolved proteins that can either have new specificities which might enhance the immunogenicity of some proteins and/or enhance the activity of the subunits once they are bound to the proteasome. Because the transition from a non-specific proteasome to a class I epitope-specific proteasome can pass through several states (in which some but not all of the interferon-inducible subunits are associated with the proteasome), many different proteolytic specificities can potentially be achieved. Evolving the specific LMP-like subunits can therefore create new proteasome compositions which have enhanced functionality for the presentation of epitopes.

The methods involve performing DNA shuffling using as substrates two or more forms of polynucleotides which encode proteasome components, where the forms of polynucleotides differ in at least one nucleotide. Shuffling is performed as described herein, using polynucleotides that encode any one or more of the various proteasome components, including, for example, LMP2, LMP7, MECL-1 and other individual proteasome components that are specifically involved in class I epitope presentation. Examples of suitable substrates are described in, *e.g.,* Stohwasser *et al.* (1997) *Eur. J. Immunol.* 27: 1182-1187 and Gaczynska *et al.* (1996) *J. Biol. Chem.* 271: 17275-17280. In a preferred embodiment, family shuffling is used, in which the different substrates are proteasome component-encoding polynucleotides from different species.

After the recombination reaction is completed, the resulting library of recombinant polynucleotides is screened to identify those which encode proteasome components having the desired effect on class I epitope production. For example, the recombinant polynucleotides can be introduced into a genetic vaccine vector which also encodes a particular antigen of interest. The library of vectors can then be introduced into mammalian cells which are then screened to identify cells which exhibit increased antigen-specific immunogenicity. Methods of analyzing proteasome activity are described in, for example, Groettrup *et al.* (1997) *Proc. Nat'l. Acad. Sci. USA* 94: 8970-8975 and Groettrup *et al.* (1997) *Eur. J. Immunol.* 26: 863-869.

Alternatively, one can use the methods of the invention to evolve proteins which bind strongly to the proteasome but have decreased or no activity, thus antagonizing the proteasome activity and diminishing a cells ability to present class I molecules. Such molecules can be applied to gene therapy protocols in which it is desirable to lower the immunogenicity of exogenous proteins expressed in the cells as a result of the gene therapy, and which would otherwise be processed for class I presentation allowing the cell to be recognized by the immune system. Such high-affinity low-activity LMP-like subunits will demonstrate immunosuppressive effects which are also of use in other therapeutic protocols where cells expressing a non-self protein need to be protected from an immune response.

The specificity of the proteasome and the TAP molecules (discussed below) may have co-evolved naturally. Thus it may be important that the two pathways of the class I processing system be functionally matched. A further aspect of the invention involves performing DNA shuffling simultaneously on the two gene families followed by random combinations of the two in order to discover appropriate matched proteolytic and transport specificities.

### 2. Antigen Transport

The invention provides methods of improving transport of antigenic peptides from the cytosolic compartment to the endoplasmic reticulum and thereby to the cell surface in the context of MHC class I molecules. Enhanced expression of antigenic peptides results in enhanced immune response, particularly in improved activation of CD8⁺ cytotoxic lymphocytes. This is useful in the development of DNA vaccines and in gene therapy.

In one embodiment, the invention involves evolving TAP-genes (transporters associated with antigen processing) to obtain genes that exhibit improved antigen presentation. TAP genes are members of ATP-binding cassette family of membrane translocators. These proteins transport antigenic peptides to MHC class I molecules and are involved in the expression and stability of MHC class I molecules on the cell surface. Two TAP genes, TAP1 and TAP2, have been cloned to date (Powis *et al.* (1996) *Proc. Nat'l. Acad. Sci. USA* 89: 1463-1467; Koopman *et al.* (1997) *Curr. Opin. Immunol.* 9: 80-88; Monaco (1995) *J. Leukocyte Biol.* 57: 543-57). TAP1 and TAP2 form a heterodimer and these genes are required for transport of peptides into the endoplasmic reticulum, where they bind to MHC class I molecules. The essential role of TAP gene products in presentation of antigenic peptides was demonstrated in mice with disrupted TAP genes. TAP1-deficient mice have drastically reduced levels of surface expression of MHC class I, and positive selection of CD8⁺ T cells in the thymus is strongly reduced. Therefore, the number of CD8+ T lymphocytes in the periphery of TAP-deficient mice is extremely low. Transfection of TAP genes back into these cells restores the level of MHC class I expression.

TAP genes are a good target for gene shuffling because of natural polymorphism and because these genes of several mammalian species have been cloned and sequenced, including human (Beck *et al.* (1992) *J Mol. Biol.* 228: 433-441; Genbank Accession No. Y13582; Powis *et al., supra.*), gorilla TAP1 (Laud *et al.* (1996) *Human Immunol.* 50: 91-102), mouse (Reiser *et al.* (1988) *Proc. Nat'l. Acad. Sci. USA* 85: 2255-2259; Marusina *et al.* (1997) *J. Immunol.* 158: 5251-5256, TAP1: Genbank Accession Nos. U60018, U60019, U60020, U60021, U60022, and L76468-L67470; TAP2: Genbank Accession Nos. U60087, U60088, U6089, U60090, U60091 and U60092), hamster (TAP1, Genbank Accession Nos. AF001154 and AF001157; TAP2, Genbank Accession Nos. AF001156 and AF001155). Furthermore, it has been shown that point mutations in TAP genes may result in altered peptide specificity and peptide presentation. Also, functional differences in TAP genes derived from different species have been observed. For example, human TAP and rat TAP containing the rTAP2a allele are rather promiscuous, whereas mouse TAP is restrictive and select against peptides with C-terminal small polar/hydrophobic or positively charged amino acids. The basis for this selectivity is unknown.

The methods of the invention involve performing DNA shuffling of TAP1 and TAP2 genes using as substrates at least two forms of TAP1 and/or TAP2 polynucleotide sequences which differ in at least one nucleotide position. In a preferred embodiment, TAP sequences derived from several mammalian species are used as the substrates for shuffling. Natural polymorphism of the genes can provide additional diversity of substrate. If desired, optimized TAP genes obtained from one round of shuffling and screening can be subjected to additional shuffling/screening rounds to obtain further optimized TAP-encoding polynucleotides.

To identify optimized TAP-encoding polynucleotides from a library of recombinant TAP genes, the genes can be expressed on the same plasmid as a target antigen of interest. If this step is limiting the extent of antigen presentation, then enhanced presentation to CD8⁺ CTL will result. Mutants of TAPs may act selectively to increase expression of a particular antigen peptide fragment for which levels of expression are otherwise limiting, or to cause transport of a peptide that would normally never be transferred into the RER and made available to bind to MHC Class I.

When used in the context of gene therapy vectors in cancer treatment, evolved TAP genes provide a means to enhance expression of MHC class I molecules on tumor cells and obtain efficient presentation of antigenic tumor-specific peptides. Thus, vectors that contain the evolved TAP genes can induce potent immune responses against the malignant cells. Shuffled TAP genes can be transfected into malignant cell lines that express low levels of MHC class I molecules using retroviral vectors or electroporation. Transfection efficiency can be monitored using marker genes, such as green fluorescent protein, encoded by the same vector as the TAP genes. Cells expressing equal levels of green fluorescent protein but the highest levels of MHC class I molecules, as a marker of efficient TAP genes, are then sorted using flow cytometry, and the evolved TAP genes are then recovered from these cells by, for example, PCR or by recovering the entire vectors. These sequences can then subjected into new rounds of shuffling, selection and recovery, if further optimization is desired.

Molecular evolution of TAP genes can be combined with simultaneous evolution of the desired antigen. Simultaneous evolution of the desired antigen can further improve the efficacy of presentation of antigenic peptides following DNA vaccination. The antigen can be evolved, using gene shuffling, to contain structures that allow optimal presentation of desired antigenic peptides when optimal TAP genes are expressed. TAP genes that are optimal for presentation of antigenic peptides of one given antigen may be different from TAP genes that are optimal for presentation of antigenic peptide of another antigen. Gene shuffling technique is ideal, and perhaps the only, method to solve this type of problems. Efficient presentation of desired antigenic peptides can be analyzed using specific cytotoxic T lymphocytes, for example, by measuring the cytokine production or CTL activity of the T lymphocytes using methods known to those of skill in the art.

### 3. Cytotoxic T-Cell Inducing Sequences And Immunogenic Agonist Sequences

Certain proteins are better able than others to carry MHC class I epitopes because they are more readily used by the cellular machinery involved in the necessary processing for class I epitope presentation. The invention provides methods of identifying expressed polypeptides that are particularly efficient in traversing the various biosynthetic and degradative steps leading to class I epitope presentation and the use of these polypeptides to enhance presentation of CTL epitopes from other proteins.

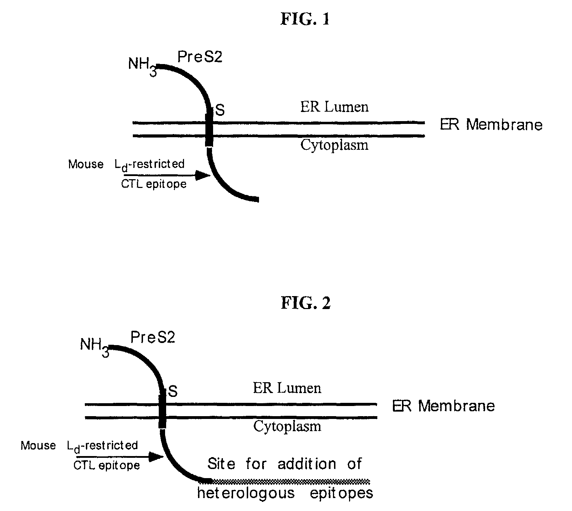

In one embodiment, the invention provides Cytotoxic T-cell Inducing Sequences (CTIS), which can be used to carry heterologous class I epitopes for the purpose of vaccinating against the pathogen from which the heterologous epitopes are derived. One example of a CTIS is obtained from the hepatitis B surface antigen (HBsAg), which has been shown to be an effective carrier for its own CTL epitopes when delivered as a protein under certain conditions. DNA immunization with plasmids expressing the HBsAg also induces high levels of CTL activity. The invention provides a shorter, truncated fragment of the HBsAg polypeptide which functions very efficiently in inducing CTL activity, and attains CTL induction levels that are higher than with the HBsAg protein or with the plasmids encoding the full-length HBsAg polypeptide. Synthesis of a CTIS derived from HBsAg is described in Example 3; and a diagram of a CTIS is shown in Figure 1.

The ER localization of the truncated polypeptide may be important in achieving suitable proteolytic liberation of the peptide(s) containing the CTL epitopes (see Cresswell & Hughes (1997) *Curr. Biol.* 7: R552-R555; Craiu *et al.* (1997) *Proc. Nat'l. Acad. Sci. USA* 94: 10850-10855). The preS2 region and the transmembrane region provide T-helper epitopes which may be important for the induction of a strong cytotoxic immune response. Because the truncated CTIS polypeptide has a simple structure (*see* Figure 1), it is possible to attach one or more heterologous class I epitope sequences to the C-terminal end of the polypeptide without having to maintain any specific protein conformation. Such sequences are then available to the class I epitope processing mechanisms. The size of the polypeptide is not subject to the normal constraints of the native HBsAg structure. Therefore the length of the heterologous sequence and thus the number of included CTL epitopes is flexible. This is shown schematically in Figure 2. The ability to include a long sequence containing either multiple and distinct class I sequences, or alternatively different variations of a single CTL sequence, allows DNA shuffling methodology to be applied.

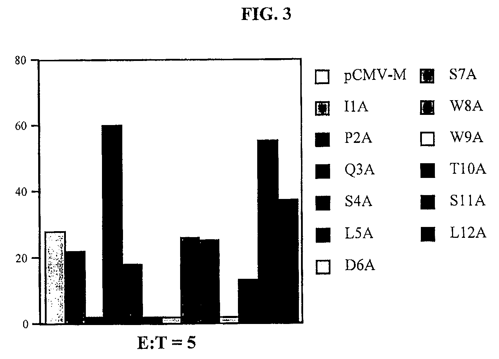

The invention also provides methods of obtaining Immunogenic Agonist Sequences (IAS) which induce CTLs capable of specific lysis of cells expressing the natural epitope sequence. In some cases, the reactivity is greater than if the CTL response is induced by the natural epitope (*see*, Example 3 and Figure 3). Such IAS-induced CTL may be drawn from a T-cell repertoire different from that induced by the natural sequence. In this way, poor responsiveness to a given epitope can be overcome by recruiting T cells from a larger pool. In order to discover such IAS, the amino acid at each position of a CTL-inducing peptide (excluding perhaps the positions of the so-called anchor residues) can be varied over the range of the 19 amino acids not normally present at the position. DNA shuffling methodology can be used to scan a large range of sequence possibilities.

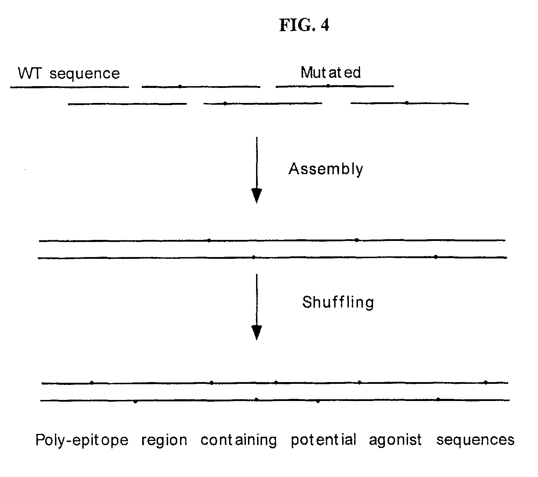

A synthetic gene segment containing multiple copies of the original epitope sequence can be prepared such that each copy possesses a small number of nucleotide changes. The gene segment can be shuffled to create a diverse range of CTL epitope sequences, some of which should function as IAS. This process is illustrated in Figure 4.

In practice, oligonucleotides are typically constructed in accordance with the above design and polymerized enzymatically to form the synthetic gene segment of the concatenated epitopes. Restriction sites can be incorporated into a fraction of the oligonucleotides to allow for cleavage and selection of given size ranges of the concatenated epitopes, most of which will have different sequences and thus will be potential IAS. The epitope-containing gene segment can be joined by appropriate cloning methods to a CTIS, such as that of HBsAg. The resulting plasmid constructions can be used for DNA-based immunization and CTL induction.

### E. Genetic Vaccine Pharmaceutical Compositions and Methods of Administration

The improved immunomodulatory polynucleotides and polypeptides of the invention are useful for treating and/or preventing the various diseases and conditions with which the respective antigens are associated. For example, genetic vaccines that employ the reagents obtained according to the methods of the invention are useful in both prophylaxis and therapy of infectious diseases, including those caused by any bacterial, fungal, viral, or other pathogens of mammals. The reagents obtained using the invention can also be used for treatment of autoimmune diseases including, for example, rheumatoid arthritis, SLE, diabetes mellitus, myasthenia gravis, reactive arthritis, ankylosing spondylitis, and multiple sclerosis. These and other inflammatory conditions, including IBD, psoriasis, pancreatitis, and various immunodeficiencies, can be treated using genetic vaccines that include vectors and other components obtained using the methods of the invention. Genetic vaccine vectors and other reagents obtained using the methods of the invention can be used to treat allergies and asthma. Moreover, the use of genetic vaccines have great promise for the treatment of cancer and prevention of metastasis. By inducing an immune response against cancerous cells, the body's immune system can be enlisted to reduce or eliminate cancer.

In presently preferred embodiments, the reagents obtained using the invention are used in conjunction with a genetic vaccine vector. The choice of vector and components can also be optimized for the particular purpose of treating allergy or other conditions. For example, an antigen associated with treating a particular condition can be optimized using recombination and selection methods analogous to those described herein. Such methods, and antigens appropriate for various conditions, are described in copending, commonly assigned US Patent Application Serial No. ________, entitled "Antigen Library Immunization," which was filed on February 10, 1999 as TTC Attorney Docket No. 18097-028710US. The polynucleotide that encodes the recombinant antigenic polypeptide can be placed under the control of a promoter, *e.g.*, a high activity or tissue-specific promoter. The promoter used to express the antigenic polypeptide can itself be optimized using recombination and selection methods analogous to those described herein, as described in International Application No. PCT/US97/17300 (International Publication No. WO 98/13487). The reagents obtained using the methods of the invention can also be used in conjunction with multicomponent genetic vaccines, which are capable of tailoring an immune response as is most appropriate to achieve a desired effect (*see, e.g.,* copending, commonly assigned US Patent Application Serial No.__________ , entitled "Genetic Vaccine Vector Engineering," filed on February 10, 1999 as TTC Attorney Docket No. 18097-030100US). It is sometimes advantageous to employ a genetic vaccine that is targeted for a particular target cell type (*e.g.*, an antigen presenting cell or an antigen processing cell); suitable targeting methods are described in copending, commonly assigned US patent application Serial No. ______, entitled "Targeting of Genetic Vaccine Vectors," filed on February 10, 1999 as TTC Attorney Docket No. 18097-030200US.

Genetic vaccine vectors that include the optimized recombinant polynucleotides obtained as described herein can be delivered to a mammal (including humans) to induce a therapeutic or prophylactic immune response. Vaccine delivery vehicles can be delivered *in vivo* by administration to an individual patient, typically by systemic administration (*e.g.*, intravenous, intraperitoneal, intramuscular, subdermal, intracranial, anal, vaginal, oral, buccal route or they can be inhaled) or they can be administered by topical application. Alternatively, vectors can be delivered to cells *ex vivo,* such as cells explanted from an individual patient (*e.g.*, lymphocytes, bone marrow aspirates, tissue biopsy) or universal donor hematopoietic stem cells, followed by reimplantation of the cells into a patient, usually after selection for cells which have incorporated the vector.

A large number of delivery methods are well known to those of skill in the art. Such methods include, for example liposome-based gene delivery (Debs and Zhu (1993) WO 93/24640; Mannino and Gould-Fogerite (1988) *BioTechniques* 6(7): 682-691; Rose U.S. Pat No. 5,279,833; Brigham (1991) WO 91/06309; and Felgner *et al.* (1987) *Proc. Natl. Acad. Sci. USA* 84: 7413-7414), as well as use of viral vectors (*e.g*., adenoviral (*see, e.g.,* Berns *et al.* (1995) *Ann. NY Acad. Sci.* 772: 95-104; Ali *et al.* (1994) *Gene Ther.* 1: 367-384; and *Haddada et al.* (1995) *Curr. Top. Microbiol. Immunol.* 199 ( Pt 3): 297-306 for review), papillomaviral, retroviral (*see, e.g.,* Buchscher *et al.* (1992) *J. Virol.* 66(5) 2731-2739; Johann *et al.* (1992) *J. Virol.* 66 (5):1635-1640 (1992); Sommerfelt *et al.,* (1990) *Virol.* 176:58-59; Wilson *et al.* (1989) *J. Virol.* 63:2374-2378; Miller et al., *J. Virol.* 65:2220-2224 (1991); Wong-Staal *et al.,* PCT/US94/05700, and Rosenburg and Fauci (1993) in *Fundamental Immunology, Third Edition* Paul (ed) Raven Press, Ltd., New York and the references therein, and Yu *et al., Gene Therapy* (1994) *supra.*)*,* and adeno-associated viral vectors (*see,* West *et al.* (1987) *Virology* 160:38-47; Carter *et al.* (1989) U.S. Patent No. 4,797,368; Carter *et al.* WO 93/24641 (1993); Kotin (1994) *Human Gene Therapy* 5:793-801; Muzyczka (1994) *J. Clin. Invst.* 94:1351 and Samulski (*supra*) for an overview of AAV vectors; *see also,* Lebkowski, U.S. Pat. No. 5,173,414; Tratschin *et al.* (1985) *Mol. Cell. Biol.* 5(11):3251-3260; Tratschin, *et al.* (1984) *Mol. Cell. Biol.,* 4:2072-2081; Hermonat and Muzyczka (1984) *Proc. Natl. Acad. Sci. USA,* 81:6466-6470; McLaughlin *et al.* (1988) and Samulski *et al.* (1989) *J. Virol.,* 63:03822-3828), and the like.

"Naked" DNA and/or RNA that comprises a genetic vaccine can be introduced directly into a tissue, such as muscle. *See, e.g.,* USPN 5,580,859. Other methods such as "biolistic" or particle-mediated transformation (*see, e.g.,* Sanford *et al.,* USPN 4,945,050; USPN 5,036,006) are also suitable for introduction of genetic vaccines into cells of a mammal according to the invention. These methods are useful not only for *in vivo* introduction of DNA into a mammal, but also for *ex vivo* modification of cells for reintroduction into a mammal. As for other methods of delivering genetic vaccines, if necessary, vaccine administration is repeated in order to maintain the desired level of immunomodulation.

Genetic vaccine vectors (*e.g.*, adenoviruses, liposomes, papillomaviruses, retroviruses, *etc*.) can be administered directly to the mammal for transduction of cells *in vivo.* The genetic vaccines obtained using the methods of the invention can be formulated as pharmaceutical compositions for administration in any suitable manner, including parenteral (*e.g.*, subcutaneous, intramuscular, intradermal, or intravenous), topical, oral, rectal, intrathecal, buccal (*e.g.*, sublingual), or local administration, such as by aerosol or transdermally, for prophylactic and/or therapeutic treatment. Pretreatment of skin, for example, by use of hair-removing agents, may be useful in transdermal delivery. Suitable methods of administering such packaged nucleic acids are available and well known to those of skill in the art, and, although more than one route can be used to administer a particular composition, a particular route can often provide a more immediate and more effective reaction than another route.

Pharmaceutically acceptable carriers are determined in part by the particular composition being administered, as well as by the particular method used to administer the composition. Accordingly, there is a wide variety of suitable formulations of pharmaceutical compositions of the present invention. A variety of aqueous carriers can be used, e.g., buffered saline and the like. These solutions are sterile and generally free of undesirable matter. These compositions may be sterilized by conventional, well known sterilization techniques. The compositions may contain pharmaceutically acceptable auxiliary substances as required to approximate physiological conditions such as pH adjusting and buffering agents, toxicity adjusting agents and the like, for example, sodium acetate, sodium chloride, potassium chloride, calcium chloride, sodium lactate and the like. The concentration of genetic vaccine vector in these formulations can vary widely, and will be selected primarily based on fluid volumes, viscosities, body weight and the like in accordance with the particular mode of administration selected and the patient's needs.

Formulations suitable for oral administration can consist of (a) liquid solutions, such as an effective amount of the packaged nucleic acid suspended in diluents, such as water, saline or PEG 400; (b) capsules, sachets or tablets, each containing a predetermined amount of the active ingredient, as liquids, solids, granules or gelatin; (c) suspensions in an appropriate liquid; and (d) suitable emulsions. Tablet forms can include one or more of lactose, sucrose, mannitol, sorbitol, calcium phosphates, corn starch, potato starch, tragacanth, microcrystalline cellulose, acacia, gelatin, colloidal silicon dioxide, croscarmellose sodium, talc, magnesium stearate, stearic acid, and other excipients, colorants, fillers, binders, diluents, buffering agents, moistening agents, preservatives, flavoring agents, dyes, disintegrating agents, and pharmaceutically compatible carriers. Lozenge forms can comprise the active ingredient in a flavor, usually sucrose and acacia or tragacanth, as well as pastilles comprising the active ingredient in an inert base, such as gelatin and glycerin or sucrose and acacia emulsions, gels, and the like containing, in addition to the active ingredient, carriers known in the art. It is recognized that the genetic vaccines, when administered orally, must be protected from digestion. This is typically accomplished either by complexing the vaccine vector with a composition to render it resistant to acidic and enzymatic hydrolysis or by packaging the vector in an appropriately resistant carrier such as a liposome. Means of protecting vectors from digestion are well known in the art. The pharmaceutical compositions can be encapsulated, *e.g.*, in liposomes, or in a formulation that provides for slow release of the active ingredient.

The packaged nucleic acids, alone or in combination with other suitable components, can be made into aerosol formulations (*e.g.*, they can be "nebulized") to be administered via inhalation. Aerosol formulations can be placed into pressurized acceptable propellants, such as dichlorodifluoromethane, propane, nitrogen, and the like.

Suitable formulations for rectal administration include, for example, suppositories, which consist of the packaged nucleic acid with a suppository base. Suitable suppository bases include natural or synthetic triglycerides or paraffin hydrocarbons. In addition, it is also possible to use gelatin rectal capsules which consist of a combination of the packaged nucleic acid with a base, including, for example, liquid triglycerides, polyethylene glycols, and paraffin hydrocarbons.

Formulations suitable for parenteral administration, such as, for example, by intraarticular (in the joints), intravenous, intramuscular, intradermal, intraperitoneal, and subcutaneous routes, include aqueous and non-aqueous, isotonic sterile injection solutions, which can contain antioxidants, buffers, bacteriostats, and solutes that render the formulation isotonic with the blood of the intended recipient, and aqueous and non-aqueous sterile suspensions that can include suspending agents, solubilizers, thickening agents, stabilizers, and preservatives. In the practice of this invention, compositions can be administered, for example, by intravenous infusion, orally, topically, intraperitoneally, intravesically or intrathecally. Parenteral administration and intravenous administration are the preferred methods of administration. The formulations of packaged nucleic acid can be presented in unit-dose or multi-dose sealed containers, such as ampoules and vials.

Injection solutions and suspensions can be prepared from sterile powders, granules, and tablets of the kind previously described. Cells transduced by the packaged nucleic acid can also be administered intravenously or parenterally.

The dose administered to a patient, in the context of the present invention should be sufficient to effect a beneficial therapeutic response in the patient over time. The dose will be determined by the efficacy of the particular vector employed and the condition of the patient, as well as the body weight or vascular surface area of the patient to be treated. The size of the dose also will be determined by the existence, nature, and extent of any adverse side-effects that accompany the administration of a particular vector, or transduced cell type in a particular patient.

In determining the effective amount of the vector to be administered in the treatment or prophylaxis of an infection or other condition, the physician evaluates vector toxicities, progression of the disease, and the production of anti-vector antibodies, if any. In general, the dose equivalent of a naked nucleic acid from a vector is from about 1 µg to 1 mg for a typical 70 kilogram patient, and doses of vectors used to deliver the nucleic acid are calculated to yield an equivalent amount of therapeutic nucleic acid. Administration can be accomplished via single or divided doses.

In therapeutic applications, compositions are administered to a patient suffering from a disease (*e.g.*, an infectious disease or autoimmune disorder) in an amount sufficient to cure or at least partially arrest the disease and its complications. An amount adequate to accomplish this is defined as a "therapeutically effective dose." Amounts effective for this use will depend upon the severity of the disease and the general state of the patient's health. Single or multiple administrations of the compositions may be administered depending on the dosage and frequency as required and tolerated by the patient. In any event, the composition should provide a sufficient quantity of the proteins of this invention to effectively treat the patient.

In prophylactic applications, compositions are administered to a human or other mammal to induce an immune response that can help protect against the establishment of an infectious disease or other condition.

The toxicity and therapeutic efficacy of the genetic vaccine vectors provided by the invention are determined using standard pharmaceutical procedures in cell cultures or experimental animals. One can determine the LD₅₀ (the dose lethal to 50% of the population) and the ED₅₀ (the dose therapeutically effective in 50% of the population) using procedures presented herein and those otherwise known to those of skill in the art.

A typical pharmaceutical composition for intravenous administration would be about 0.1 to 10 mg per patient per day. Dosages from 0.1 up to about 100 mg per patient per day may be used, particularly when the drug is administered to a secluded site and not into the blood stream, such as into a body cavity or into a lumen of an organ. Substantially higher dosages are possible in topical administration. Actual methods for preparing parenterally administrable compositions will be known or apparent to those skilled in the art and are described in more detail in such publications as *Remington's Pharmaceutical Science,* 15th ed., Mack Publishing Company, Easton, Pennsylvania (1980).

The multivalent antigenic polypeptides of the invention, and genetic vaccines that express the polypeptides, can be packaged in packs, dispenser devices, and kits for administering genetic vaccines to a mammal. For example, packs or dispenser devices that contain one or more unit dosage forms are provided. Typically, instructions for administration of the compounds will be provided with the packaging, along with a suitable indication on the label that the compound is suitable for treatment of an indicated condition. For example, the label may state that the active compound within the packaging is useful for treating a particular infectious disease, autoimmune disorder, tumor, or for preventing or treating other diseases or conditions that are mediated by, or potentially susceptible to, a mammalian immune response.

### EXAMPLES

The following examples are offered to illustrate, but not to limit the present invention.

### Example 1

### Altered ligand specificity of B7-1 (CD80) and/or B7-2 (CD86) by DNA shuffling

This Example describes the use of the DNA shuffling methods of the invention to obtain B7-1 and B7-2 polypeptides that have altered biological activities.

### DNA shuffling

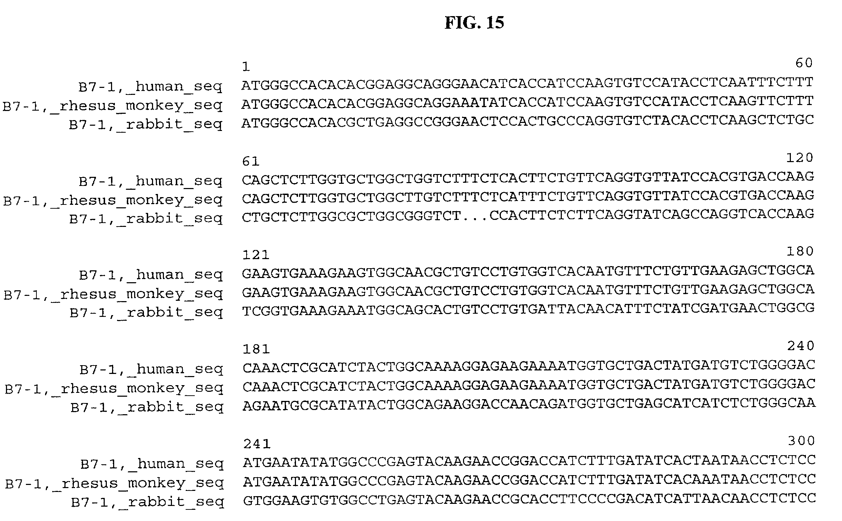

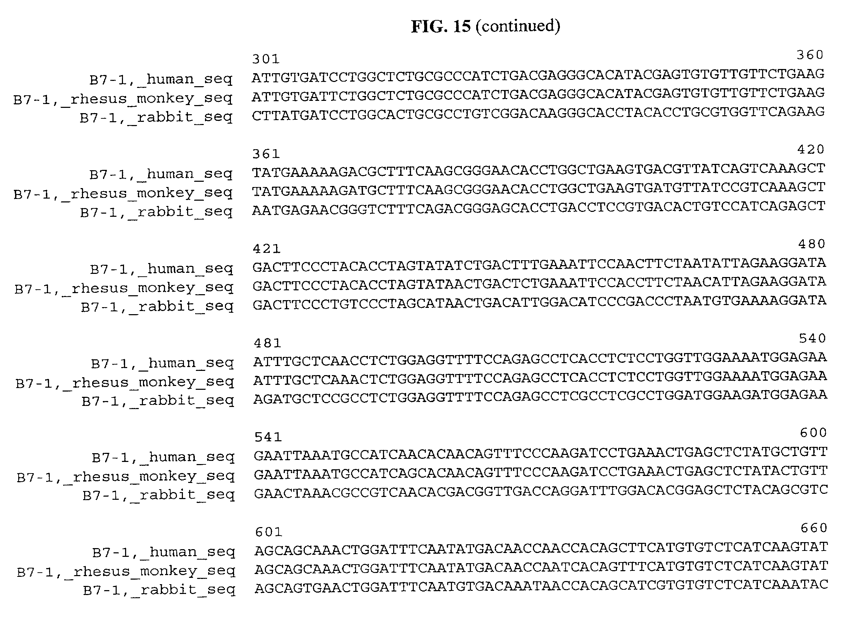

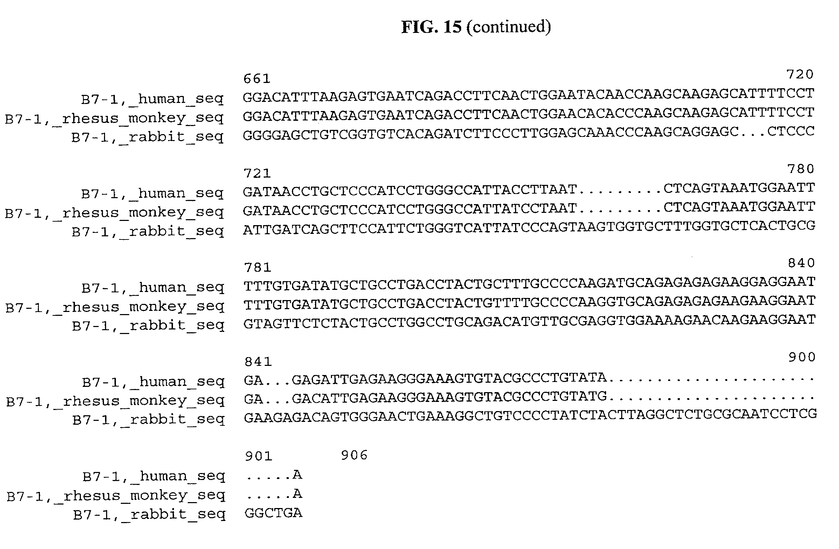

DNA shuffling is used to generate a library of B7 (B7-1/CD80 and B7-2/CD86) variants that have altered relative capacity to act through CD28 and CTLA-4 when compared to wild-type B7 molecules. Typically, B7 cDNAs from various species are generated by RT-PCR, and these sequences are shuffled using family DNA shuffling. Alignments of human, rhesus monkey and rabbit B7-1 nucleotide sequences are shown in Figure 15, demonstrating that family DNA shuffling is a feasible approach when evolving B7 molecules.

### Screening of B7 variants

The library is then screened to identify those variants that are useful in modulating immune responses in autoimmune diseases, allergy, cancer, infectious disease and vaccination. Any of several approaches for screening of the variants can be used:

### A. Flow cytometry-based selection system.

The library of B7-1 and B7-2 molecules is transfected into cells that normally do not express these molecules (*e.g.*, COS-7 cells or any cell line from different species with limited or no cross-reactivity with man regarding B7 ligand binding). An internal marker gene can be incorporated in order to analyze the copy number per cell.

Soluble CTLA-4 and CD28 molecules are generated to facilitate the flow cytometry experiments. Typically, these soluble polypeptides are fused with the Fc portion of IgG molecule to improve the stability of the molecules and to enable easy staining by labeled anti-IgG monoclonal antibodies, as described by van der Merwe *et al.* ((1997) *J*. *Exp. Med.* 185: 393). The cells transfected with the library of B7 molecules are then stained with the soluble CTLA-4 and CD28 molecules. Cells demonstrating increased or decreased CTLA-4/CD28 binding ratio are sorted. The plasmids are then recovered and the shuffled sequences identified. These selected B7 variants can then be subjected to new rounds of shuffling and selection, and can be further analyzed using functional assays as described below.

### B. Selection based on functional properties.

Bacterial colonies that contain plasmids that include mutant B7 molecules are picked and the plasmids are isolated. These plasmids are then transfected into antigen presenting cells, such as dendritic cells, and the capacities of these mutants to activate T cells is analyzed. One of the advantages of this approach is that no assumptions are made as to the binding affinities or specificities to the known ligands, and possibly new activities through yet to be identified ligands can be found.

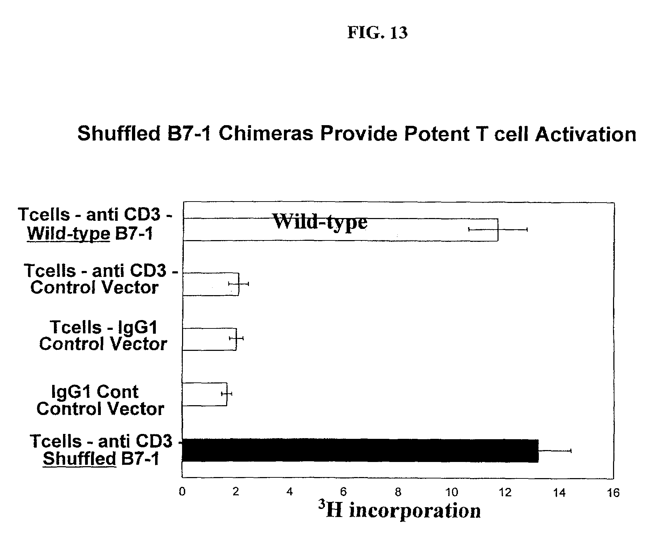

T cell activation can be analyzed by measuring proliferation, cytokine production, CTL activity or expression of activation antigens such as IL-2 receptor, CD69 or HLA-DR molecules. Usage of antigen-specific T cell clones, such as T cells specific for house dust mite antigen Der p I, allows analysis of antigen-specific T cell activation. Mutants are identified that can enhance or inhibit T cell proliferation or enhance or inhibit CTL responses. Similarly variants that have altered capacity to induce cytokine production or expression of activation antigens as measured by, for example, cytokine-specific ELISAs or flow cytometry can be selected. Results obtained using a proliferation-based assay is shown are shown in Figure 13.

### C. Ability to direct either T_{H}1 or T_{H}2 cell differentiation.

Because differential roles for B7-1 and B7-2 molecules in the regulation of T helper cell differentiation have been identified (Freeman *et al.* (1995) *Immunity* 2: 523; Kuchroo *et al.* (1995) *Cell* 80: 707), one can screen for B7 variants that are the most effective in directing either T_{H}1 or T_{H}2 cell differentiation. T_{H} cell differentiation can be measured by analyzing the cytokine production profiles induced by each particular variant. High levels of IL-4, IL-5 and /or IL-13 are an indication of efficient T_{H}2 cell differentiation whereas high levels of IFN-γ or IL-2 production can be used as a marker of T_{H}1 cell differentiation. B7 variants that altered capacity to induce T_{H}1 or T_{H}2 cell differentiation are likely to be useful in the treatment of allergic, malignant, autoimmune and infectious diseases and in vaccination.

### D. Enhanced IL-10 production.

Elevated production of IL-10 is a characteristic of regulatory T cells, which can suppress proliferation of antigen-specific CD4⁺ T cells (Groux *et al.* (1997) *Nature* 389: 737). Therefore, B7 variants can be screened to identify those that have enhanced capacity to induce IL-10 production by antigen-specific T cells. IL-10 production can be measured, for example, by ELISA or flow cytometry using intracytoplasmic cytokine stainings. The variants that induce high levels of IL-10 production are useful in the treatment of allergic and autoimmune diseases.

### Example 2

### Evolution Of Cytokines For Improved Specific Activity And/Or Improved Expression Levels

This example describes a method to evolve a cytokine for improved specific activity and/or improved expression levels when the genetic vaccine is transfected into mammalian cells. IL-12 is the most potent cytokine directing T_{H}1 responses, and it improves the efficacy of genetic vaccinations. Evolved IL-12 molecules are useful as components of genetic vaccines. IL-12 is a heterodimeric cytokine composed of a 35 kD light chain (p35) and a 40 kD heavy chain (p40) (Kobayashi *et al.* (1989) *J. Exp. Med.* 170: 827; Stern *et al.* (1990) *Proc. Nat'l. Acad. Sci. USA* 87: 6808). Recently Lieschke *et al.* (*Nature Biotechnol.* (1997) 15: 35) demonstrated that fusion between p35 and p40 genes results in a single gene that has activity comparable to that of the two genes expressed separately. Accordingly, an IL-12 gene is shuffled as one entity that encodes both subunits, which is beneficial in designing the shuffling protocol. The subunits of IL-12 can also be expressed separately in the same expression vector, or the subunits can be expressed separately and screened using cotransfections of the two vectors, providing additional shuffling strategies.

IL-12 plays several roles in the regulation of allergic responses. For example, IL-12 induces T_{H}1 cell differentiation and downregulates the T_{H}2 response. IL-12 inhibits IgE synthesis both *in vivo* and *in vitro,* and also induces IFN-γ production. Accordingly, it is desirable to obtain an optimized IL-12 that better able to carry out these functions upon administration to a mammal.

Cytokine genes, including IL-12 genes, from humans and nonhuman primates are generally 93-99% homologous (Villinger *et al.* (1995) *J. Immunol.* 155:3946-3954), providing a good starting point for family shuffling. A library of shuffled IL-12 genes was obtained by shuffling p35 and p40 subunits derived from human, rhesus monkey, cat, dog, cow, pig, and goat, and incorporated into vectors and the supernatants of these transfectants are analyzed for biological activity as shown in Figure 6. Because of its T cell growth promoting activities, it is possible to use normal human peripheral blood T cells in the selection of the most active IL-12 genes, enabling directly to select IL-12 mutants with the most potent activities on human T cells.

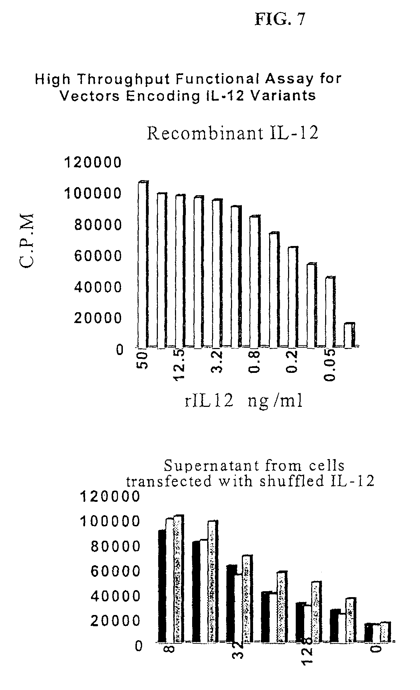

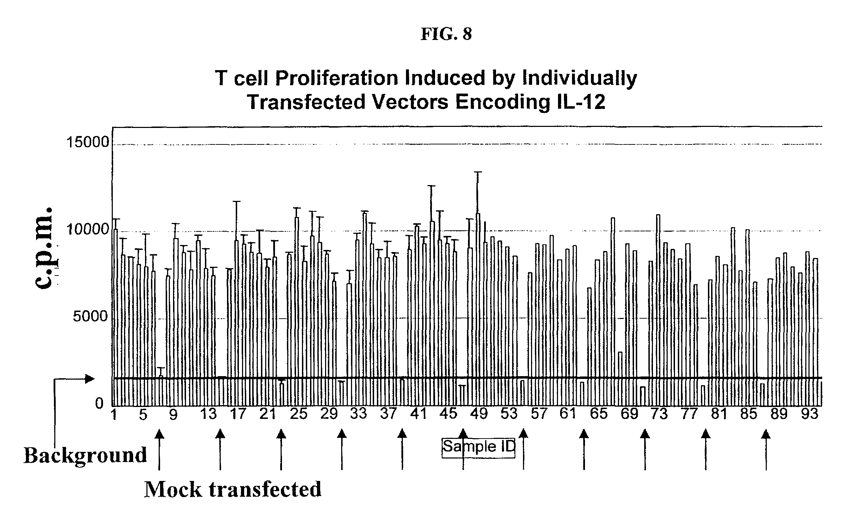

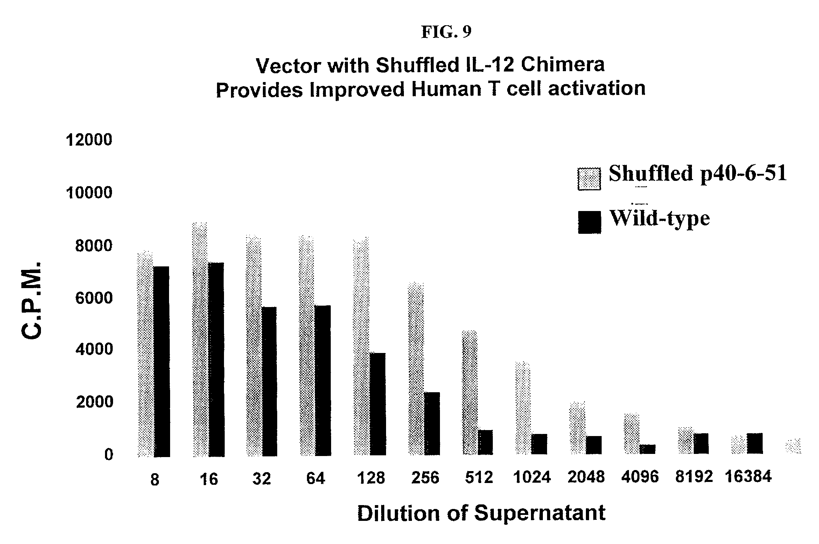

As shown in Figure 7, a functional screening assay has been successfully established. In this assay, COS-7 cells were first transfected with vectors encoding IL-12 subunits. Forty-eight hours after transfection, the capacity of these culture supernatants to induce proliferation of activated human peripheral blood T cells was studied. Figure 8 indicates the consistency of the level of T cell proliferation induced in this assay, indicating that the assay can be used to distinguish the activities between supernatants that have different capacities to induce T cell activation. In other words, the assay provides means to screen for improved IL-12-like activities in culture supernatants of transfected cells. A vector with an optimized IL-12-encoding polynucleotide was tested for ability to induce human T cell activation. Results, shown in Figure 9, show that the shuffled IL-12 has an significantly increased ability to induce T cell activation compared to wild-type IL-12.

Figure 6 illustrates a general strategy for screening of evolved cytokine genes. The specific example is given for IL-12 but similar approach applies to all cytokines when using cell types sensitive for each cytokine. For example, GM-CSF can be evolved by the same approach by using the GM-CSF sensitive cell line TF-1 in the screening. In addition, although in this example the vectors are transfected into CHO cells, any mammalian cell that can be transfected in vitro can be used as host cells. In addition to CHO cell, other good host cells include cell lines WI-26, COS-1, COS-7, 293, U937 and freshly isolated human antigen presenting cells, such as monocytes, B cells and dendritic cells.

### Example 3

### Cytotoxic T-cell Inducing Sequences Derived from Hepatitis B Surface Antigen and Strongly Immunogenic Agonistic T cell Epitopes

This Example describes the preparation of a polypeptide sequence capable of efficient presentation of T cell epitopes and a strategy for the application of DNA shuffling to discover strongly immunogenic agonistic T cell epitopes.

The HBsAg polypeptide (PreS2 plus S regions) was truncated by the introduction of a stop codon at amino acid position 103 (counting from the beginning of the PreS2 initiator methionine), transforming a cysteine codon TGT into the Stop codon TGA. The amino acid sequence of the truncated protein was therefore: 
MQWNSTTFHQTLQDPRVRGLYFPAGGSSSGTVNPVLTTASPLSSIFSRIGDPALNME
NITSGFLGPLLVLQAGFFLLTRILTIPQSLDSWWTSLNFLGGTTV*
 where the standard single-letter code for amino acids is used. The methionine residues at the start of the PreS2 and S regions are underlined, and the mouse L^{d}-restricted CTL epitope is double-underlined; the asterisk (*) represents the artificially introduced Stop codon.

A likely structure for this truncated polypeptide is shown in Figure 1. During protein biosynthesis, the N-terminal region of the HBsAg polypeptide is transported through the membrane of the endoplasmic reticulum (ER). The first part of the S region is a transmembrane structure which locks the polypeptide into the ER. The remaining C-terminal region of the polypeptide is located in the cytoplasmic compartment where it can be accessed by the epitope processing mechanism of the cell. This structure forms what is referred to as the Cytotoxic T-cell Inducing Sequence (CTIS).

A CTIS is preferably used in conjunction with an immunogenic agonistic sequence (IAS). As an example of an IAS, each position of a 12 amino acid L^{d}-restricted class I epitope from HBsAg was replaced with an alanine-encoding codon in the DNA sequence of the epitope. The results demonstrate that, in some cases, the reactivity is greater than if the CTL response is induced by the natural epitope (Figure 3).

### Example 4

### Treatment of Obesity, Anorexia, and Cachexia using Optimized Immunomodulatory Molecules

Optimized immunomodulatory molecules that are obtained using the methods of the invention find use in a wide variety of applications, in addition to use in vaccination. For example, there is increasing evidence that certain forms of obesity are associated with dysfunction of the immune system, and that molecules which regulate immune responses, *e.g.* cytokines, can induce or inhibit obesity. The invention provides methods of optimizing immune regulatory molecules for the treatment of obesity, anorexia and cachexia.

Leptin and ciliary neurotrophic factor (CNTF) are examples of cytokines that have been shown to play role in the development of obesity. Congenital leptin deficiency results in severe early-onset obesity in human (Montague *et al.* (1997) *Nature* 387: 903-908), and CNTF has been shown to correct obesity and diabetes associated with leptin deficiency and resistance (Gloaguen *et al.* (1997) *Proc. Nat'l. Acad. Sci. USA* 94: 6456-6461). Antagonists of CNTF and/or leptin may be useful in the treatment of anorexia and/or cachexia.

The methods of the invention are used to generate leptin and/or CNTF molecules that have improved specific activity. The methods are also useful for obtaining improved cytokines that exhibit reduced immunogenicity *in vivo*; immunogenicity is a particular concern for CNTF, because the wild-type CNTF is highly antigenic, which results in the production of high levels of anti-CNTF antibodies when administered to a human. Improved cytokine molecules prepared using the methods of the invention are administered as polypeptides, or the shuffled nucleic acids that encode improved leptin and/or CNTF polypeptides are used in genetic vaccine vectors. The invention also provides methods of generating vectors that induce production of increased levels of leptin and/or CNTF.

The methods of the invention can also be used to obtain reagents that are useful for the treatment of anorexia, cachexia, and related disorders. In this embodiment, antagonists of leptin and/or CNTF are evolved using the DNA shuffling methods. For example, a leptin receptor can be evolved to obtain a soluble form that has an enhanced affinity for leptin. The receptor for leptin in mice is found in the hypothalamus (Mercer *et al.* (1996) *FEBS Lett.* 387: 113), a region known to be involved in maintenance of energy balance, and in the choroid plexus and leptomeninges, which form part of the blood/brain barrier.

It is understood that the examples and embodiments described herein are for illustrative purposes only and that various modifications or changes in light thereof will be suggested to persons skilled in the art and are to be included within the spirit and purview of this application and scope of the appended claims. All publications, patents, and patent applications cited herein are hereby incorporated by-reference for all purposes.

## Claims

1. A method for obtaining a polynucleotide that has a modulatory effect on an immune response, or encodes a polypeptide that has a modulatory effect on an immune response, that is induced by a genetic vaccine vector, the method comprising:
creating a library of recombinant polynucleotides; and
screening the library to identify an optimized recombinant polynucleotide that has, or encodes a polypeptide that has, a modulatory effect on an immune response induced by a genetic vaccine vector;
wherein the optimized recombinant polynucleotide or the polypeptide encoded by the recombinant polynucleotide exhibits an enhanced ability to modulate an immune response compared to a non-recombinant polynucleotide from which the library was created.

2. The method of claim 1, wherein the optimized recombinant polynucleotide is incorporated into a genetic vaccine vector.

3. The method of claim 1, wherein the optimized recombinant polynucleotide, or a polypeptide encoded by the optimized recombinant polynucleotide, is administered in conjunction with a genetic vaccine vector.

4. The method of claim 1, wherein the library of recombinant polynucleotides is created by a process selected from the group consisting of DNA shuffling, error-prone PCR, oligonucleotide-directed mutagenesis, uracil-mediated mutagenesis, and repair-deficient host mutagenesis.

5. The method of claim 1, wherein the polynucleotide that has a modulatory effect on an immune response is obtained by:
(1) recombining at least first and second forms of a nucleic acid that is, or encodes a molecule that is, involved in modulating an immune response, wherein the first and second forms differ from each other in two or more nucleotides, to produce a library of recombinant polynucleotides; and
(2) screening the library to identify at least one optimized recombinant polynucleotide that exhibits, either by itself or through the encoded molecule, an enhanced ability to modulate an immune response than a form of the nucleic acid from which the library was created.

6. The method of claim 5, wherein the method further comprises the steps of:
(3) recombining at least one optimized recombinant polynucleotide with a further form of the nucleic acid, which is the same or different from the first and second forms, to produce a further library of recombinant polynucleotides;
(4) screening the further library to identify at least one further optimized recombinant polynucleotide that exhibits an enhanced ability to modulate an immune response than a form of the nucleic acid from which the library was created.; and
(5) repeating (3) and (4), as necessary, until the further optimized recombinant polynucleotide exhibits an further enhanced ability to modulate an immune response than a form of the nucleic acid from which the library was created.

7. The method of claim 1, wherein the optimized recombinant polynucleotide encodes a peptide or polypeptide that can interact with a cellular receptor involved in mediating an immune response, wherein the peptide or polypeptide acts as an agonist or antagonist of the receptor.

8. The method of claim 7, wherein the cellular receptor is a macrophage scavenger receptor.

9. The method of claim 7, wherein the cellular receptor is selected from the group consisting of a cytokine receptor and a chemokine receptor.

10. The method of claim 9, wherein the chemokine receptor is CCR6.

11. The method of claim 7, wherein the peptide or polypeptide mimics the activity of a natural ligand for the receptor but does not induce immune reactivity to the natural ligand.

12. The method of claim 7, wherein the library is screened by:
expressing the recombinant polynucleotides so that the encoded peptides or polypeptides are produced as fusions with a protein displayed on the surface of a replicable genetic package;
contacting the replicable genetic packages with a plurality of cells that display the receptor; and
identifying cells that exhibit a modulation of an immune response mediated by the receptor.

13. The method of claim 12, wherein the replicable genetic package is selected from the group consisting of a bacteriophage, a cell, a spore, and a virus.

14. The method of claim 13, wherein the replicable genetic package is an M13 bacteriophage and the protein is encoded by geneIII or geneVII.

15. The method of claim 7, which method further comprises introducing the optimized recombinant polynucleotide into a genetic vaccine vector and administering the vector to a mammal, wherein the peptide or polypeptide is expressed and acts as an agonist or antagonist of the receptor.

16. The method of claim 7, which method further comprises producing the peptide or polypeptide encoded by the optimized recombinant polynucleotide and introducing the peptide or polypeptide into a mammal in conjunction with a genetic vaccine vector.

17. The method of claim 7, wherein the optimized recombinant polynucleotide is inserted into an antigen-encoding nucleotide sequence of a genetic vaccine vector.

18. The method of claim 17, wherein the optimized recombinant polypeptide is introduced into a nucleotide sequence that encodes an M-loop of an HBsAg polypeptide.

19. The method of claim 1, wherein the optimized recombinant polynucleotide comprises a nucleotide sequence rich in unmethylated CpG.

20. The method of claim 1, wherein the optimized recombinant polynucleotide encodes a polypeptide that inhibits an allergic reaction.

21. The method of claim 20, wherein the polypeptide is selected from the group consisting of interferon-α, interferon-γ, IL-10, IL-12, an antagonist of IL-4, an antagonist of IL-5, and an antagonist of IL-13.

22. The method of 1, wherein the optimized recombinant polynucleotide encodes an antagonist of IL-10.

23. The method of claim 22, wherein the antagonist of IL-10 is soluble or defective IL-10 receptor or IL-20/MDA-7.

24. The method of claim 1, wherein the optimized recombinant polynucleotide encodes a costimulator.

25. The method of claim 24, wherein the costimulator is B7-1 (CD80) or B7-2 (CD86) and the screening step involves selecting variants with altered activity through CD28 or CTLA-4.

26. The method of claim 24, wherein the costimulator is CD1, CD40, CD154 (ligand for CD40) or CD150 (SLAM).

27. The method of claim 24, wherein the costimulator is a cytokine.

28. The method of claim 27, wherein the cytokine is selected from the group consisting of IL-1, IL-2, IL-3, IL-4, IL-5, IL-6, IL-7, IL-8, IL-9, IL-10, IL-11, IL-12, IL-13, IL-14, IL-15, IL-16, IL-17, IL-18, GM-CSF, G-CSF, TNF-α, IFN-α, IFN-γ, and IL-20 (MDA-7).

29. The method of 28, wherein the library of recombinant polynucleotides is screened by testing the ability of cytokines encoded by the recombinant polynucleotides to activate cells which contain a receptor for the cytokine.

30. The method of claim 29, wherein the cells contain a heterologous nucleic acid that encodes the receptor for the cytokine.

31. The method of 28, wherein the cytokine is interleukin-12 and the screening is performed by:
growing mammalian cells which contain the genetic vaccine vector in a culture medium; and
detecting whether T cell proliferation or T cell differentiation is induced by contact with the culture medium.

32. The method of 28, wherein the cytokine is interferon-α and the screening is performed by:
expressing the recombinant polynucleotides so that the encoded peptides or polypeptides are produced as fusions with a protein displayed on the surface of a replicable genetic package;
contacting the replicable genetic packages with a plurality of B cells; and
identifying phage library members that are capable of inhibiting proliferation of the B cells.

33. The method of claim 28, wherein the immune response of interest is differentiation of T cells to T_{H}1 cells and the screening is performed by contacting a population of T cells with the cytokines encoded by the members of the library of recombinant polynucleotides and identifying library members that encode a cytokine that induces the T cells to produce IL-2 and interferon-γ.

34. The method of claim 27, wherein the cytokine encoded by the optimized recombinant polynucleotide exhibits reduced immunogenicity compared to a cytokine encoded by a non-optimized polynucleotide, and the reduced immunogenicity is detected by introducing a cytokine encoded by the recombinant polynucleotide into a mammal and determining whether an immune response is induced against the cytokine.

35. The method of claim 24, wherein the costimulator is B7-1 (CD80) or B7-2 (CD86) and the cell is tested for ability to costimulate an immune response.

36. The method of claim 1, wherein the optimized recombinant polynucleotide encodes a cytokine antagonist.

37. The method of claim 36, wherein the cytokine antagonist is selected from the group consisting of a soluble cytokine receptor and a transmembrane cytokine receptor having a defective signal sequence.

38. The method of claim 36, wherein the cytokine antagonist is selected from the group consisting of ΔIL-10R and ΔIL-4R.

39. The method of claim 1, wherein the optimized recombinant polynucleotide encodes a polypeptide capable of inducing a predominantly T_{H}1 immune response.

40. The method of claim 1, wherein the optimized recombinant polynucleotide encodes a polypeptide capable of inducing a predominantly T_{H}2 immune response.

41. A method for obtaining a polynucleotide that encodes an accessory molecule that improves the transport or presentation of antigens by a cell, the method comprising:
creating a library of recombinant polynucleotides by subjecting to recombination nucleic acids that encode all or part of the accessory molecule; and
screening the library to identify an optimized recombinant polynucleotide that encodes a recombinant accessory molecule that confers upon a cell an increased or decreased ability to transport or present an antigen on a surface of the cell compared to an accessory molecule encoded by the non-recombinant nucleic acids.

42. The method of claim 41, wherein the screening involves:
introducing the library of recombinant polynucleotides into a genetic vaccine vector that encodes an antigen to form a library of vectors;
introducing the library of vectors into mammalian cells; and
identifying mammalian cells that exhibit increased or decreased immunogenicity to the antigen.

43. The method of claim 41, wherein the accessory molecule comprises a proteasome or a TAP polypeptide.

44. The method of claim 41, wherein the accessory molecule comprises a cytotoxic T-cell inducing sequence.

45. The method of claim 44, wherein the cytotoxic T-cell inducing sequence is obtained from a hepatitis B surface antigen.

46. The method of claim 41, wherein the accessory molecule comprises an immunogenic agonist sequence.
